# EUROPEAN PATENT APPLICATION

(11) **EP 2 156 798 A2**
(43) Date of publication of application: **24.02.2010**
(21) Application number: 09010746.7
(22) Date of filing: 20.08.2009
(51) Int. Cl.: A61B 17/128, A61B 17/08, A61B 17/122

(54) **Clip package and clip loading method**

(30) Priority: 21.08.2008 JP 2008212663
(71) Applicant: FUJIFILM Corporation, Minato-ku Tokyo (JP)
(72) Inventor: Itoh, Koji, Kanagawa (JP); Iida, Takayuki, Kanagawa (JP); Kunuki, Yoshiyuki, Kanagawa (JP)
(74) Representative: Höhfeld, Jochen

(57) **Abstract**

A clip package comprises: a clip assembly including at least one endoscopic clip and at least one connection ring connected to the clip, the connection ring having at least one skirt portion that is allowed to expand radially outwards and stick out when in natural state free from external force and closes inwards when depressed radially inwards, a cylindrical case including an accommodation section for accommodating the clip assembly, the cylindrical case further including at least one opening in a position corresponding to the skirt portion of the connection ring, the opening being provided to maintain the skirt portion in natural state, and a cylindrical outer tube movably fitted over the outer periphery of the case and including at least one skirt closer for depressing and inwardly closing the skirt portion when the skirt closer is moved to a position opposite the opening.

## Description

### BACKGROUND OF THE INVENTION

The present invention relates to a package for distribution and storage of an endoscopic clip used for hemostasis, closure of a diseased part such as a wound, etc. in a living body or the like and, in particular, to a clip package for a successive clipping device that permits a successive use of clips.

The present invention also relates to a method of loading the clip from the clip package into a sheath.

An endoscopic clipping device is used to cause a clip to protrude from the tip of an endoscope inserted into a living body and pinch a bleeding site or a region to be treated after the removal of a lesion tissue, thereby achieving hemostasis, closure of a diseased part such as a wound, and the like. The conventional clipping device comprises a clip having open ends and a clamping portion for closing and placing the clip. Advancing the clamping portion in relation to the open clip closes the clip.

For example, US 2002/0133178 A1 describes a configuration wherein a clip clamping ring is attached to the rear end of a clip and wherein the clip is pulled relative to the clip clamping ring to retract the clip into the clip clamping ring and close the clippers at the tip of the clip, thus pinching a body tissue of interest. The clip clamping ring described in US 2002/0133178 A1 is pushed out together with the clip when the clip is pushed out from the tip of a guide tube (sheath). The clip clamping ring comprises two retractable blades, which open as they pass by the chip attached to the tip of the sheath so that the clip clamping ring thereafter does not retract into the sheath even if the clip is pulled backwards. Pulling the clip in such a state causes the clip clamping ring to tighten the clip. Subsequently, the clip clamping ring is placed in a living body together with the clip.

In a conventionally used endoscopic clipping device including the clipping device described in US 2002/0133178 A1, only a single clip is attached to the forward end of a manipulating wire. Therefore, each time clipping is performed, the entire sheath is withdrawn from the endoscope, and the sheath is loaded with the next clip before being inserted into the endoscope again for the next clipping. Thus, the conventional clipping device involves a rather bothersome operation.

In contrast thereto, endoscopic clipping devices allowing successive clipping were proposed. For example, JP 2006-187391A describes an endoscopic clipping device comprising a plurality of clips directly connected with each other in such a positional relationship that the connection hole formed at the rear end of one clip engages with the claw portion provided at the forward end of the following clip and the clips are alternately twisted 90° with respect to the adjacent ones.

In the clipping device described in US 2002/0133178 A1 and conventional clipping devices, the clip is typically housed in the clip case in storage or distribution and, for use, is withdrawn from the clip case, connected to the clip manipulating wire, which is so disposed as to be able to move forward and backward in the sheath of the clipping device, and loaded into the sheath. Then, the sheath of the clipping device is inserted into the endoscope located in a living body to position the sheath at the tip of the endoscope, and the clip is allowed to stick out from the tip to perform hemostasis, closure of a wound, etc. in a bleeding region or a treated region after removal of a lesion tissue.

Accordingly, there has been proposed a clip case facilitating the connection of the clip housed in the clip case and the manipulating wire disposed in the sheath of the clipping device and the loading of the clip in the clip case into the sheath of the clipping device.

For example, JP 2007-222649A proposes a clip case housing a clip unit composed of a clip, a holder tube (tightening ring) fitted on the clip to close the clip, and a connection member that can be inserted into the holder tube so as to engage with the clip and has an arrow head hook on the opposite side from the clip.

The clip case proposed in JP 2007-222649A comprises an accommodation section, a large bore section, and a small bore section. The accommodation section houses a clip unit formed of a plate member where the protuberances of the holder tube of the connection member are projecting. The large bore section is formed opposite the arrow head hook of the connection member of the clip unit inside the accommodation section and has a diameter larger than the outer diameter of the coil pipe of the clip operating device. The small bore section communicates with the large bore section and has a diameter smaller than the inner diameter of the coil pipe that is formed on the side closer to the accommodation section but larger than the outer diameter of the holder tube. The clip case has a slant portion formed between the accommodation section and the small bore section.

According to the description in JP 2007-222649A, the slant portion provided between the accommodation section and the small bore section serves to depress said projections of the holder tube as the clip unit is withdrawn from the clip case, permitting smooth insertion of the clip unit into the clipping device (into the coil pipe and the coil sheath in the sheath).

However, the clip case proposed in JP 2007-222649A had a problem: retraction of the projections into the holder tube is achieved by abutting the projections of the clip holder tube against the slant portion formed close to the exit of the clip case, whereas the direction in which the projections are inclined with respect to the clip's moving direction is opposite to the direction in which the slant portion is formed, thus placing a load upon the projections (skirt portion) and possibly breaking the projections.

The clipping device described in JP 2006-187391A is capable of successive clipping. However, since the clip case described in JP 2007-222649A houses only one clip, the clip case described in JP 2007-222649A cannot be applied to the clipping device described in JP 2006-187391A.

### SUMMARY OF THE INVENTION

An objection of the present invention is to provide a clip package that eliminates the above problems and permits distribution and storage with an endoscopic clip and a connection ring coupled to each other, easy loading into the sheath of the endoscopic clip while keeping such connection, and easy application to also a clipping device that performs successive clipping.

Another object of the invention is to provide a clip loading method of loading a clip from such a clip package into a sheath.

A clip package according to the present invention comprises: a clip assembly including at least one endoscopic clip and at least one cylindrical connection ring connected to the at least one endoscopic clip, the connection ring having at least one skirt portion that is allowed to expand radially outwards and stick out when in natural state free from external force and closes inwards when depressed radially inwards, a cylindrical case including an accommodation section for accommodating the clip assembly, the cylindrical case further including at least one opening in a position corresponding to the skirt portion of the connection ring of the clip assembly accommodated in the accommodation section, the at least one opening being provided to maintain the skirt portion in natural state, and a cylindrical outer tube movably fitted over the outer periphery of the case and including at least one skirt closer for depressing and inwardly closing the skirt portion when the at least one skirt closer is moved to a position opposite the at least one opening.

A clip loading method according to the present invention of loading a sheath for an endoscopic clipping device with the clip assembly accommodated in the case of such a clip package, comprising the steps of: connecting a forward end of a manipulating wire provided in the sheath for withdrawing the clip assembly to a connection member attached to a rearmost end of the clip assembly accommodated in the case, connecting the forward end of the sheath and the proximal end of the case, placing the case and the outer tube in their relative positions to cause the at least one skirt closer to close the at least one skirt portion, and moving the manipulating wire and the sheath relative to each other in a direction in which the forward end of the manipulating wire moves toward the proximal end of the sheath so that the whole of the clip assembly is loaded in the sheath.

### BRIEF DESCRIPTION OF THE DRAWINGS

FIGS. 1A and 1B are a side sectional view and a front sectional view illustrating a successive clipping device used in the invention, respectively.
FIG. 2 is a perspective view illustrating a configuration of a clip used in the clipping device of FIG. 1.
FIGS. 3A through 3C are a front view, a front sectional view, and a bottom view, respectively, illustrating a connection ring used in the clipping device of FIG. 1.
FIGS. 4A and 4B are a partial plan sectional view and a partial front sectional view, respectively, schematically illustrating a configuration of a manipulating unit used in the clipping device of FIG. 1.
FIGS. 5A through 5E are partial sectional views respectively illustrating progressive stages of the successive clipping device of FIG. 1 in its clipping operation.
FIGS. 6A through 6C are a side view, a side sectional view, and a front sectional view, respectively, illustrating a successive clip package according to a first embodiment of the invention.
FIG. 7 is a partial enlarged view of FIG. 6B.
FIGS. 8A through 8D are partial sectional views respectively illustrating progressive stages of a loading operation whereby the clip unit is loaded from the successive clip package into the sheath in the first embodiment.
FIGS. 9A through 9C are a side view, a side sectional view, and a front sectional view, respectively, illustrating a successive clip package according to a second embodiment.
FIGS. 10A through 10D are partial sectional views respectively illustrating progressive stages of the loading operation whereby the clip unit is loaded from the successive clip package into the sheath in the second embodiment.
FIGS. 11A through 11C are a perspective view, a side sectional view, and a front sectional view, respectively, illustrating a successive clip package according to a third embodiment.
FIGS. 12A and 12B are partial sectional views respectively illustrating the successive clipping package of the third embodiment in its different states in use.
FIGS. 13A through 13C are a side view, a side sectional view, and a front sectional view, respectively, illustrating a successive clip package according to a fourth embodiment.
FIGS. 14A and 14B are a side sectional view and a front sectional view respectively illustrating the successive clip package of the fourth embodiment at the time of storing.
FIGS. 15A and 15B are a side sectional view and a front sectional view illustrating the successive clip package of the fourth embodiment at the time of loading.
FIGS. 16A to 16D are partial sectional views illustrating relationships between a skirt portion and a flap of the connected clip package in respective variations of a fifth embodiment.

### DETAILED DESCRIPTION OF THE INVENTION

A clip package and a clip loading method according to the present invention are described in detail based on the embodiments thereof illustrated in the accompanying drawings.

First, a successive clipping device will be described.

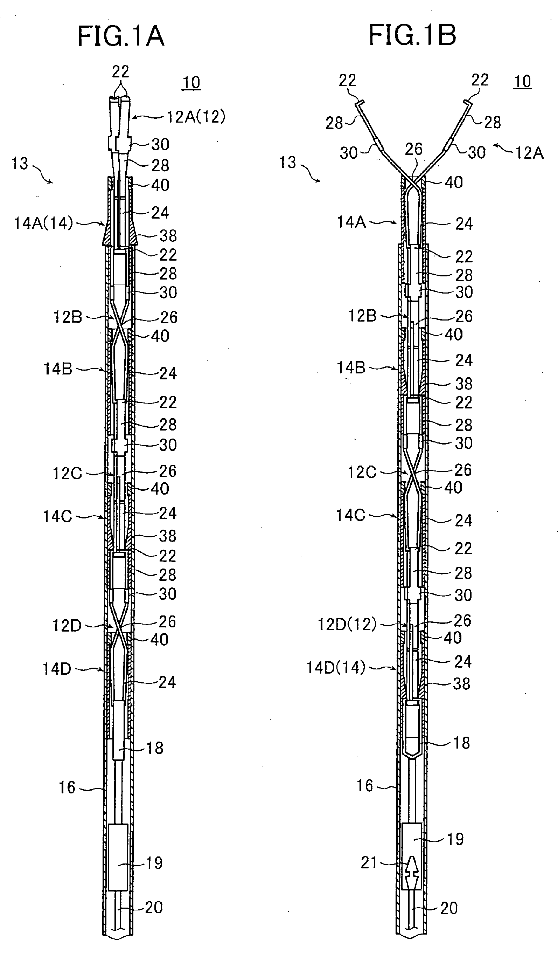

FIGS. 1A and 1B illustrate a schematic configuration of a successive clipping device (hereinafter also referred to simply as "clipping device") 10.

In the following description, an upper end portion in each of FIGS. 1A and 1B is called a "forward end", and a lower end portion in each of FIGS. 1A and 1B is called a "proximal end".

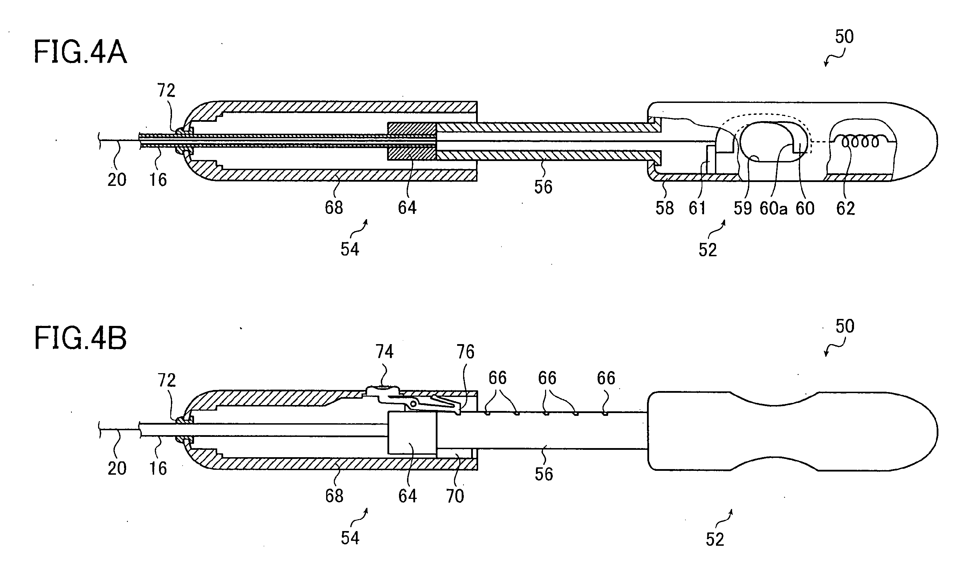

The clipping device 10 includes a plurality of clips 12 (12A, 12B, 12C and 12D), connection rings 14 (14A, 14B, 14C and 14D) that cover the engagement portions of the adjacent clips 12 to maintain the connection states of the clips 12, a sheath 16 into which the clips 12 and the connection rings 14 are fitted, a dummy clip 18 connected to the rearmost fourth clip 12D, a manipulating wire 20 connected to the dummy clip via a connecting member 19, and a manipulating portion 50 (see FIGS 4A and 4B) attached to the proximal end of the sheath 16 for manipulating the manipulating wire 20.

The plurality of clips 12 (12A, 12B, 12C and 12D), the connection rings 14 (14A, 14B, 14C and 14D), the rearmost dummy clip 18, and the connecting member 19 constitute a clip series (connected clip unit) 13. The dummy click 18 is a member for connecting the manipulating wire 20 and the plurality of clips 12 via the connecting member 19.

FIGS. 1A and 1B illustrate an initial state (standby state) immediately before an operation of clipping manipulation using the foremost clip 12A is started.

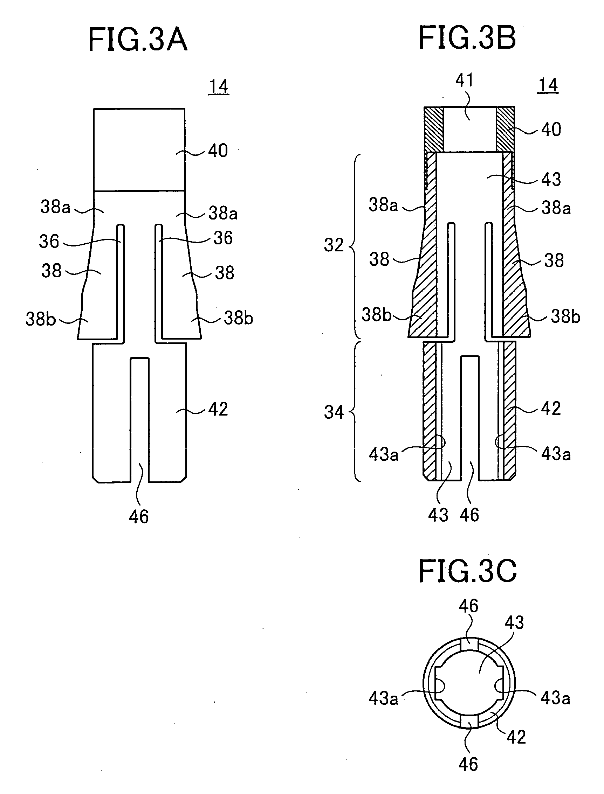

One of the clips 12 and the corresponding one of the connection rings 14 including a clamping ring 40 (see FIGS 3A and 3B) described later constitute an endoscopic bleeding stop clip unit. The clipping device 10 includes a plurality of such bleeding stop clip units loaded inside the forward end of the elongated sheath 16.

The terminal end of the consecutive bleeding stop clip units are engagedly connected to the dummy clip 18 as a member for connecting the clip series 13 and the manipulating wire 20. The dummy clip 18 is connected to the manipulating wire 20 via the connecting member 19. The manipulating wire 20 extends to the proximal end portion of the sheath 16, and connected to the manipulating portion 50 (see FIGS. 4A and 4B) described later.

By pulling the manipulating wire 20 from the manipulating portion 50 by a predetermined pulling length, and moving the dummy clip 18 in one direction by a predetermined distance, a series of the clips 12 move by equal amounts, and the foremost clip 12 is clamped by the clamping ring 40 at the forward end of the connection ring 14 retaining the foremost clip 12 to effect clipping manipulation (clipping) for stopping bleeding, marking, or the like by the foremost clip 12. After the clipping manipulation using the foremost clip 12 is completed, by pushing the manipulating wire 20 toward the forward end of the sheath 16 by a predetermined length, the next clip 12 is brought into a usable state (standby state) to allow clipping manipulation to be performed in succession.

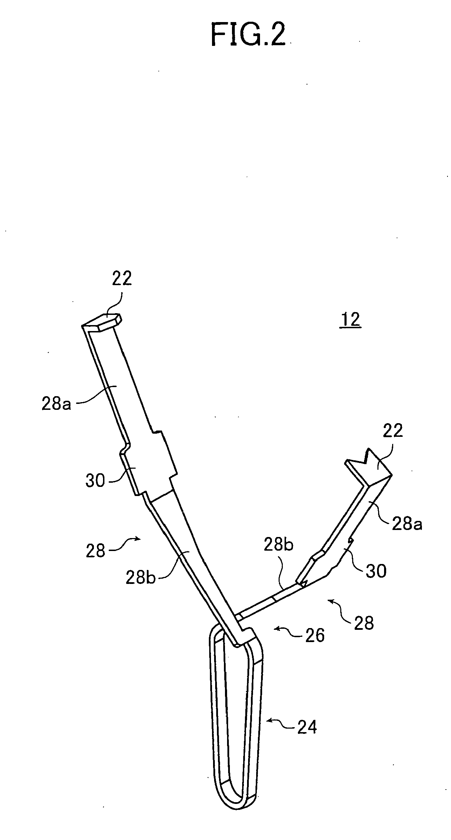

FIGS. 1A and 1B are views of a state in which the foremost first clip 12A has protruded from the forward end of the sheath 16, but when the clips 12 and the like are loaded in the sheath 16, they are set with the foremost first clip 12A being completely contained in the sheath 16. Although FIGS. 2A and 2B illustrate the four clips 12, and a four-shots clipping device, the number of the clips 12 may be any as long as it is not less than 2.

FIG. 2 is a perspective view illustrating a schematic configuration of each of the clips 12. The clip 12 is a closed clip including a turned portion 24 which is 180-degree turned with respect to claw portions 22. That is, the clip 12 has a shape obtained by bending a single elongated plate by 180 degrees to form a closed end, crossing the both ends thereof, and bending the end portions into opposing relation to form the claw portions 22 at two open ends. On the open-end side of the resulting crossing portion 26, there are arm portions 28, while there is the turned portion 24 on the closed-end side thereof. In the respective center portions of the arm portions 28, partially widened projections 30 are formed. Each of the arm portions 28 is divided by the projection 30 into a distal portion 28a closer to the claw portion 22, and a proximal portion 28b closer to the crossing portion 26. For the clips 12, a metal with biocompatibility is preferably used. For example, it is possible to use SUS 630 or SUS 631, which is a precipitation-hardening stainless steel.

The clamping ring 40 fixed to the forward end portion of the connection ring 14 fitted over the crossing portion 26 of the clip 12 moves by a predetermined amount toward the claw portions 22 (toward the projections 30), while pressing the proximal portions 28b of the arm portions 28, whereby the arm portions 28 and the claw portions 22 are closed, with the claw portions 22 exerting a predetermined fit-engagement force (gripping force).

To reliably pinch a target portion such as a bleeding portion or a portion to be subjected to manipulation after the removal of a lesion tissue, the claw portions 22 are formed into a V-shaped male type and a female type.

The widths of the distal portions 28a of the arm portions 28 of the clips 12 remain constant and invariable from the claw portions 22 to the projections 30, while the widths of the proximal portions 28b thereof gradually increase from the crossing portion 26 toward the projections 30 to be constant in the vicinities of the projections 30. This facilitates and ensures the movement of the clamping ring 40 as well as the opening, closing, and fit-engagement of the claw portions 22, thereby facilitating and ensuring the stopping of bleeding, the suture or closing of a wound, and the like in a living body or the like.

The projections 30 have widths larger than the respective inner diameters of a forward-end-side opening (see a hole 41 of the clamping ring 40 in FIG. 3B, which is described later) of the connection ring 14 and a proximal-end-side opening (a hole 43 in a retaining portion 42 described later) thereof, i.e., larger than the widths of portions which come in contact with the projections 30. As a result, the portions of the clip 12 other than the projections 30 can enter the interior of the connection ring 14, but the projections 30 cannot enter the interior either from the forward-end side or proximal-end side of the connection ring 14.

As illustrated in FIGS. 1A and 1B, the claw portions 22 of the second clip 12B are engaged with the turned portion 24 of the first clip 12A, and retained in a closed state by the connection ring 14A, whereby the first clip 12A and the second clip 12B are brought into a connection state. As illustrated in FIG. 1A, the claw portions 22 of the second clip 12B orthogonally mesh with the turned portion 24 of the first clip 12A to be connected thereto, and hence the first clip 12A and the second clip 12B are connected in 90-degree different orientations. Likewise, the second clip 12B and the third clip 12C are also connected in 90-degree different orientations. Further, the third clip 12C and the fourth clip 12D are also connected in 90-degree different orientations.

Each of the connection rings 14 is fitted into the sheath 16 so as to be capable of advancing and retreating, while covering the engagement portion between the two preceding and subsequent clips 12, and maintaining a connection state therebetween. That is, the outer diameter of the connection ring 14 is slightly smaller than the inner diameter of the sheath 16 to allow the connection ring 14 to smoothly advance and retreat in the sheath 16 with the movement of the clips 12. FIGS. 3A to 3C illustrate a schematic configuration of an embodiment of the connection ring, of which FIG. 3A is a front view of the connection ring 14, FIG. 3B is a cross-sectional view thereof, and FIG. 3C is a bottom view thereof.

As illustrated in FIGS. 3A to 3C, each of the connection rings 14 includes the clamping ring 40, and the retaining portion 42. The connection ring 14 has the clamping ring 40 made of metal, and fastened to the forward end of the retaining portion 42 made of resin to integrally include the two members. The retaining portion 42 made of resin serves to maintain the connection state, and retain the clip 12 in the connection ring 14, while the clamping ring 40 made of metal serves to clamp the clip 12. The connection ring 14 may be formed of a single member as long as it can perform the respective functions of both the clamping ring 40 and the retaining portion 42.

The clamping ring 40 is a cylindrical (ring-shaped) metal component mounted on the forward end of the connection ring 14, and has the hole 41 whose inner diameter is larger than the width of the clip 12 in the vicinity of the crossing portion 26, and smaller than the width of each of the projections 30. As a result, the clamping ring 40 can move in the vicinity of the crossing portion 26 of the clip 12 retained thereby, but cannot go beyond the projections 30 toward the forward end of the clip 12. That is, the projections 30 function as a stopper which determines the movement limit of the connection ring 14 advancing with respect to the clip 12.

The clamping ring 40 is set at a predetermined position in the vicinity of the crossing portion 26 of the clip 12. The clamping ring 40 moves from the initial position thereof, i.e., from the crossing portion 26 toward the projections 30, with the arm portions 28 of the clip 12 increasing in width, so as to perform a clamping function of closing both the diverged arm portions 28 of the clip 12, and fixing them. For the clamping ring 40, a metal with biocompatibility is used. For example, a stainless steel SUS 303 or SUS 304, a titanium alloy, or the like can be used. By forming the clamping portion 40 of metal, a frictional force serving as a clamping force can be exerted on the metal clip 12.

The retaining portion 42 is a substantially cylindrical (ring-shaped) component formed by resin molding. The retaining portion 42 has a first region 32 retaining the preceding clip 12, and a second region 34 which is a connection retaining region for retaining the subsequent clip 12 in a state connected to the preceding clip. The retaining portion 42 has the hole 43 connecting to the hole 41 of the clamping ring 40, and extending through the first region 32 and the second region 34.

The first region 32 is provided with the circular hole 43 capable of accommodating the turned portion 24 of the clip 12, and the proximal portions 28b of the arm portions 28 thereof, and larger than the hole 41 of the clamping ring 40. The outer surface of the forward end portion of the first region 32 is provided with a stepped portion for allowing the clamping ring 40 to be fitted. The clamping ring 40 and the retaining portion 42 are fit-engaged by such close fit as to prevent disengagement therebetween in a state in which they are loaded in the sheath 16 and during clipping manipulation.

The first region 32 has a skirt portion 38 which inclinedly diverges into a skirt-like configuration with respect to the axis of the main body of the connection ring 14.

In the skirt portion 38, the forward-end side, i.e., an upper base portion 38a in FIGS. 3A and 3B is connected to a main body 42a of the retaining portion 42, and a lower diverging portion 38b is partially disconnected from the main body 42a by a cut 36 formed in the main body 42a to be radially diverged or closed. The skirt portion 38 includes two skirt portions formed at two locations on both sides which are apart from each other by 180 degrees and at the same position in the pulling direction of the clips 12, i.e., in the vertical direction of FIGS. 4A and 4B.

As illustrated in FIG. 3A, in a natural state in which no external force is imparted thereto, both the skirt portions 38 are diverged into a skirt-like configuration. At this time, the interior of the first region 32 of the retaining portion 42 forms a columnar space, as illustrated in FIG. 3B. On the other hand, when the connection ring 14 is loaded into the sheath 16, as in the case with, e.g., the second connection ring 14B illustrated in FIG. 1B, the skirt portions 38 (diverging portions 38b thereof) are inwardly pushed to enter the internal space, and the inner-circumferential-side portions of the skirt portions 38 (diverging portions 38b thereof) press the side surface (edge portion) of the turned portion 24 of the second clip 12B retained by the first region 32 to retain and keep the second clip 12B from moving in a rotating direction and an advancing/retreating direction within the connection ring 14B. The skirt portions 38 may press and retain the clip retained by the second region 34, i.e., the rear-side clip.

As in the case with the first connection ring 14A illustrated in FIG. 1A, on coming out from the forward end of the sheath 16, the skirt portions 38 are simultaneously opened due to their own elasticity, thus releasing the first clip 12A from retention, and becoming wider than the inner diameter of the sheath 16 to prevent the connection ring 14A from retreating into the sheath 16. In this state, the manipulating wire 20 is pulled, and the first clip 12A retreats, whereby the connection ring 14A advances relatively to the first clip 12A to clamp the first clip 12A with the clamping ring 40 integrally fastened to the connection ring 14A.

Therefore, it is necessary for the skirt portions 38 to have elasticity so as to be capable of being inwardly closed within the sheath 16, and diverged into a skirt-like configuration when they get out of the forward end of the sheath 16 and released from an external force. At the same time, it is also necessary for the skirt portions 38 to have rigidity which enables the clip 12 to be retained within the sheath 16, and rigidity which withstands the repulsive force of the clamping force of the clip 12 at the forward end of the sheath 16.

From those viewpoints, for the retaining portion 42, a material having biocompatibility and providing elasticity and rigidity each required of the skirt portions 38 is used. In addition, the shape thereof is determined so as to provide elasticity and rigidity required of the skirt portions 38. Examples of a material that can be used for the retaining portion 42 include polyphenylsulfone (PPSU), aromatic nylon, and the like. In terms of ease of production, the retaining portion 42 is preferably molded integrally.

The second region 34 is provided on the proximal-end side of the first region 32. The second region 34 retains the next clip 12 engaged with the clip 12 retained by the first region 32, specifically the claw portions 22 and the distal portions 28a of the arm portions 28 in a state in which the claw portions 22 are closed with the closed end (tail portion) of the turned portion 24 of the preceding clip 12 being held therebetween.

The second region 34 has a length which is substantially equal to the movement distance required for the clamping ring 40 set at the initial position with respect to the clip 12 to move till completing the clamping of the clip 12 as a region length. That is, while the clip 12 retreats relatively to the connection ring 14 to be clamped, the second region 34 of the connection ring 14 maintains the connection between the two clips 12 retained therein to allow the pulling force of the rear clip 12 to be transmitted to the forward-end-side clip 12 and, when the clamping by the clamping ring 40 is completed, the engagement portion of the two clips 12 exits the second region 34, thereby disconnecting the clips 12.

As illustrated in FIGS. 3B and 3C, the second region 34 is provided with the hole 43 having the same inner diameter as in the first region 32, and extending therethrough from the first region 32. The second region 34 further has two grooves (recesses) 43a formed in the two opposing portions thereof. The second region 34 also has two slits 46 that are cut from the proximal end thereof.

The grooves 43a can accommodate therein the distal portions 28a of the arm portions 28 of the clip 12 retained by the second region 34, with the claw portions 22 being closed.

The grooves 43a are provided at two positions on both sides in the direction in which the claw portions 22 of the clip 12 retained by the second region 34 are opened and closed (lateral direction in FIG. 3B). Plate surfaces of the distal portions 28a of the arm portions 28 of the clip 12 retained by the second region 34 come in contact with inner walls of the grooves 43a. The width (opening width) of each of the grooves 43a is slightly larger than the maximum width of the distal portion 28a of each of the arm portions 28 of the clip 12. The distance from the wall surface of one of the grooves 43a to the wall surface of the other groove 43a is substantially equal to the sum total of the lengths (lengths in the diverging direction) of the two claw portions 22 of the clip 12. The width of the groove 43a is smaller than the width of each of the projections 30 formed in the arm portions 28. Therefore, the projections 30 of the clip 12 retained by the second region 34 cannot enter the grooves 43a.

The arrangement can prevent the subsequent clip 12 (e.g., 12C) from overlapping the preceding clip 12 (e.g., 12B) from behind. As a result, it is possible to (1) maintain the relative positions of the anterior and posterior (preceding and subsequent) clips 12, and (2) maintain a manipulation of pushing out the clip 12 by the manipulating wire 20.
(1) When the overlapping of the subsequent clip 12 from behind occurs, a manipulation stroke of the manipulating wire 20 changes disadvantageously. However, it is possible to prevent such overlapping and maintain the relative positions of the preceding and subsequent steps 12, and hence the manipulation stroke can be maintained.
   In addition, the bending rigidity of the second region 34 has been reduced by the slit 46 provided in the connection ring 14, and angle suitability has been provided by chain-like connection between the clips 12. When the posterior clip 12 excessively comes into the anterior connection ring 14, flexibility decreases to degrade the angle suitability. However, it is possible to prevent the overlapping from behind and maintain the relative positions of the preceding and subsequent clips 12, and hence the angle suitability can be maintained.
(2) The manipulation of pushing out the clip 12 by the manipulating wire 20 can be maintained.

### <Manipulation of Pushing out Clip 12 by Manipulating Wire 20>

The projections 30 of the dummy clip 18 push the proximal-end side (the end portion of the retaining portion 42) of the connection ring 14D of the fourth clip 12D.

In the sheath 16, the fourth clip 12D has been integrated with the connection ring 14D by frictional contact so that the pushing force transmitted to the connection ring 14D is transmitted to the clip 12D (At this time, in the fourth clip 12D, the diverging portions 38b of the skirt portions 38 of the connection ring 14D are inwardly deformed to retain the turned portion 24 of the fourth clip 12D).

The projections 30 of the fourth clip 12D push the proximal-end side (the end portion of the retaining portion 42) of the connection ring 14C of the third clip 12C.

In the sheath 16, the third clip 12C has been integrated with the connection ring 14C by frictional contact so that the pushing force transmitted to the connection ring 14C is transmitted to the clip 12C.

The projections 30 of the third clip 12C push the proximal-end side (the end portion of the retaining portion 42) of the connection ring 14B of the second clip 12B.

In the sheath 16, the second clip 12B has been integrated with the connection ring 14B by frictional contact so that the pushing force transmitted to the connection ring 14B is transmitted to the clip 12B.

The projections 30 of the second clip 12B push the proximal-end side (the end portion of the retaining portion 42) of the connection ring 14A of the first clip 12A.

In the sheath 16, the first clip 12A has been integrated with the connection ring 14A by frictional contact so that the pushing force transmitted to the connection ring 14A is transmitted to the clip 12A to push it out.

The pulling force of the manipulating wire 20 is directly exerted on the clip 12, and hence it is not particularly affected by the overlapping from behind.

### <Transmission of Pulling Manipulation by Manipulating Wire 20 Effected by Clip 12>

The forward end (claw portions 22) of the dummy clip 18 pulled by the manipulating wire 20 pulls the proximal end (turned portion 24) of the fourth clip 12D.

The forward end (claw portions 22) of the fourth clip 12D pulls the proximal end (turned portion 24) of the third clip 12C.

The forward end (claw portions 22) of the third clip 12C pulls the proximal end (turned portion 24) of the second clip 12B.

The forward end (claw portions 22) of the second clip 12B pulls the proximal end (turned portion 24) of the first clip 12A.

The distance between the respective wall surfaces of the two grooves 43a may be adjusted appropriately to a dimension which does not cancel the engagement between the turned portion 24 of the preceding clip 12 and the claw portions 22 of the next clip 12, and the distance may be set smaller than the sum total of the lengths of the two claw portions 22 and the width of the portion of the turned portion 24 engaged with the claw portions 22.

For example, the claw portions 22 of the clip 12 retained by the second region 34 may be in a slightly overlapping state, or the connection of the clip with the preceding clip 12 may be maintained in a state in which the claw portions 22 have a slight gap therebetween.

The engagement portion between the two clips 12 is retained by the portion of the second region 34 close to the boundary between the second region 34 and the first region 32. Inside the sheath 16, the turned portion 24 of the preceding clip 12 (e.g., the second clip 12B in the connection ring 14B of FIG. 1B) is retained by the closed skirt portions 38 in the first region 32 so that the advancing/retreating movement and the rotating movement of the clip are restrained. The next clip 12 (e.g., the third clip 12C in the connection ring 14B of FIG. 1B) engaged with the preceding clip 12 is retained by the grooves 43a of the second region 34 in a direction 90-degrees different from the direction in which the preceding clip is retained, whereby the rotating movement thereof is restrained. The next clip 12 is also engaged with the preceding clip 12 whose advancing/retreating movement has been restrained, whereby the advancing/retreating movement thereof is restrained. That is, the engagement portion between the preceding and subsequent clips is retained in a state with very little play by the connection ring 14.

The slits 46 are formed at two positions 90-degree shifted from the skirt portions 38 so as to be shallower than the upper end of the second region 34. In other words, the slits 46 are provided at positions 90-degree shifted from the diverging direction of the clips 12 retained by the second region 34.

The provision of the slits 46 can improve the flexibility of the connection ring 14 to allow the clipping device 10 to pass through a curved portion with small curvature. The provision of the slits 46 also allows a bottom edge (proximal end portion) of the connection ring 14 to be partially turned up. Therefore, when the preceding and subsequent clips 12 are connected together prior to the loading of the clips 12 into the sheath 16, easy connection can be made advantageously by turning up the bottom edge of the connection ring 14.

The depths of the slits 46 are restricted to positions within the second region 34 not reaching the skirt portions 38 of the first region 32, thereby preventing a significant reduction in the strength of the connection ring 14. The depths of the slits 46 are also restricted to positions shallower than the position of the rear end of the clip 12 retained by the first region 32, i.e., to positions shallower than the engagement position of the clips 12. As a result, also in the connection clip unit before being loaded into the sheath 16, it is possible to maintain the retention of the clip 12 by the second region 34 of the connection ring 14.

As illustrated in FIGS. 1A and 1B, the claw portions 22 of the second clip 12B are engaged with the turned portion 24 of the first clip 12A, and the resulting engagement portion is retained by the connection ring 14A. The claw portions 22 of the second clip 12B are retained in the closed state by the inner wall of the connection ring 14A (the second region 34 thereof). As a result, the connection state between the first clip 12A and the second clip 12B is maintained. Likewise, the connection state between the second clip 12B and the third clip 12C is maintained by the connection ring 14B, the connection state between the third clip 12C and the fourth clip 12D is maintained by the connection ring 14C, and the connection state between the fourth clip 12D and the dummy clip 18 is maintained by the connection ring 14D.

The rearmost fourth clip 12D is engaged with the dummy clip 18, which is not used for clipping manipulation. The dummy clip 18 includes, at the forward end portion thereof, a resilient portion having a shape similar to that of the open-end-side half portion of the clip 12 extending from the crossing portion 26. That is, the resilient portion of the dummy clip 18 has a shape in which the arm portions including the two claw portions are connected at the crossing portion. The dummy clip 18 is engaged with the turned portion of the fourth clip 12D with the claw portions being closed, and releases the fourth clip 12D when the claw portions are opened. In the arm portions of the dummy clip 18 in the illustrated example, projections are not provided, but projections may also be provided therein. To the proximal end portion of the dummy clip 18, the connecting member 19 is attached. The connecting member 19 is detachably connected to the hook-shaped connected member (hook) 21 at the forward end of the manipulating wire 20, which is described later.

For example, the sheath 16 is a flexible coil sheath formed of a tightly wound metal wire. The sheath 16 allows the clip 12 to be movably fitted therein on the forward-end side to accommodate therein the manipulating wire 20 connected to the clip 12 via the dummy clip 18 and the connecting member 19. The sheath 16 is connected to the manipulating portion 50 on the proximal-end side. The inner diameter of the sheath 16 is adjusted to a dimension which allows canceling of the engagement between the turned portion 24 of the preceding clip 12 and the claw portions 22 of the next clip 12. That is, the inner diameter of the sheath 16 is larger than the sum total of the lengths of the two claw portions 22 and the width of the portion of the turned portion 24 engaged with the claw portions 22.

The manipulating wire 20 causes the plurality of clips 12 to advance or retreat in a series of clipping manipulations. The manipulating wire 20 is formed of, e.g., a metal wire, accommodated in the sheath 16, and includes the connected member 21 provided at the forward end portion thereof (the end portion on the opposite side of the manipulating portion 50). The forward end portion of the manipulating wire 20 is connected by the connected member 21 to the clip 12 via the connecting member 19 and the dummy clip 18, while the proximal end portion thereof to which the connected member 21 is not attached is connected to the manipulating portion 50. As described above, the proximal end portion of the sheath 16 is also attached, together with the manipulating wire 20, to the manipulating portion 50 described later.

The connected member 21 of the manipulating wire 20 and the connecting member 19 of the dummy clip 18 have shapes which allow one of the members to be fitted into the other member, and prevent disengagement therebetween when the clip 12 and the manipulating wire 20 perform an advancing/retreating operation, i.e., when they move in the sheath 16. Specifically, the connected member 21 of the manipulating wire 20 is a hook-shaped member and the connecting member 19 is provided with a connecting ring. By fitting the hook-shaped connected member 21 of the manipulating wire 20 to the connecting ring of the connecting member 19, the manipulating wire 20 can be connected with the connecting member 19. The connected member 21 and the connecting member 19 may be configured such that the connected member 21 of the manipulating wire 20 has a protruding shape (a conical shape having a protrusion on the forward-end side in this embodiment) larger in diameter than the manipulating wire 20, while the connecting member 19 has a shape in which a space substantially equal to or slightly larger than the connected member 21 is formed, and includes an opening having a diameter smaller than that of the space, and larger than that of the manipulating wire 20 is formed on the side of the space closer to the manipulating portion 50. It is to be noted that the space formed in the connecting member 19 has a shape which does not entirely circumferentially cover the side surface (surface facing the inner circumferential surface of the sheath 16) of the connected member 21. The space is opened in one direction or two opposing directions of four circumferential directions.

By thus forming the connected member 21 into a shape to be fitted to the connecting ring or into the space of the connecting member 19, and fitting the connected member 21 into the space of the connecting member 19, even when the manipulating wire 20 is pulled toward the manipulating portion 50, the hooked portion of the connected member 21 closer to the manipulating portion 50 is fitted to the connecting ring of the connecting member 19, or the surface of the connected member 21 closer to the manipulating portion 50 is supported by the surface of the connecting member 19 provided with the opening. Therefore, it is possible to prevent the connected member 21 from being disengaged from the connecting member 19.

The proximal ends of the manipulating wire 20 and the sheath 16 are attached to the manipulating portion.

FIGS. 4A and 4B are a partial plan cross-sectional view and a partial front cross-sectional view, respectively, illustrating the configuration of the manipulating portion 50 used in the clipping device. In FIGS. 4A and 4B, the left side corresponds to the forward end side connected to the clipping device 10, and the right side corresponds to the rear end side (or the proximal end side) to be manipulated by an operator.

A manipulating portion 50 includes a wire manipulating handle 52 constituting a manipulating portion main body, and a sheath manipulating handle 54 serving as a grasping portion for grasping the proximal end portion of the sheath 16, with the sheath manipulating handle 54 being slidable with respect to the wire manipulating handle 52.

The wire manipulating handle 52 includes a cylindrical case 58, a positioning pipe 56 fixed coaxially to the forward end of the case 58, and a lever 60 and a spring 62 retained inside the case 58.

The lever 60 is retained inside the case 58 so as to be movable in the longitudinal direction (axial direction of the wire manipulating handle 52). A Part of a rear end side of the lever 60 appears through a window 59 provided at the center portion of the case 58. The operator can place his/her finger onto a part of the lever 60 on the rear end side to pull the lever 60 to the rear end side. The spring 62 is attached to the rear end of the lever 60.

The spring 62 is compressed by pulling the lever 60 rearwards, and when the pulling force on the lever 60 is released, the spring 62 forwardly pushes back the lever 60 by repulsive force. As a result, the lever 60 is restored to the former position (home position).

The rearward movement limit for the lever 60 is determined by the window 59. That is, the position where a surface 60a of the lever 60 onto which the finger is hooked coincides with the rear end of the window 59 is the rearward movement limit for the lever 60. It is also possible to provide a regulating plate at the rear of the lever 60, and to determine the rearward movement limit for the lever 60 through abutment of the rear end of the lever 60 against the regulating plate.

A regulating plate 61 is provided in front of the lever 60 to determine the home position of the lever 60. Therefore, when the lever 60 is pulled to the rear end side, the lever 60 is urged by the spring 62 and moves forwards until it abuts the regulating plate 61 to return to the home position.

While in FIG. 4A the spring 62 is formed of a coil spring, this should not be construed restrictively. It is only necessary for the spring 62 to be capable of forwardly urging the lever 60. Thus, it is also possible to use a plate spring or some other elastic member as urging means.

Fixed to the forward end of the lever 60 is the manipulating wire 20 for pulling the clips 12. The manipulating wire 20 extends through the sheath manipulating handle 54 and the positioning pipe 56 to reach the lever 60.

When the operator inserts his/her finger into the window 59 and pulls the lever 60 to move the lever 60 rearwards, the manipulating wire 20 attached to the forward end of the lever 60 also moves, and the forward end of the manipulating wire 20 moves rearwards. When the pulling force applied to the lever 60 is canceled and the lever 60 is restored to the former position, the manipulating wire 20 also moves, with its forward end returning to the former position.

The pulling amount of the manipulating wire 20 in the clipping is a very small amount, e.g., 3.1 mm. Thus, in order to give a reliable operational feel at the manipulating portion 50, a pulling amount magnifying mechanism for the manipulating wire 20 may be provided between the pulling amount of the manipulating wire 20 and the manipulating amount of the lever 60, making the movement amount of the lever 60 a predetermined number of times of the movement amount of the manipulating wire 20.

The positioning pipe 56 is a hollow pipe-like member constituting a slide mechanism which allows a sheath manipulating handle 54 to move forwards and rearwards with respect to the wire manipulating handle 52, and through which the manipulating wire 20 passes. The inner diameter of the positioning pipe 56 is larger than the outer diameter of the sheath 16, making it possible to insert the sheath 16 into the positioning pipe 56.

Here, as illustrated in FIG. 4B, a plurality of (6 in the illustrated example) notches 66 (66a through 66f) used for pulling the sheath 16 by a predetermined length are formed in the upper surface of the positioning pipe 56.

The forward end portion of the positioning pipe 56 is inserted into the sheath manipulating handle 54, and a detachment prevention ring 64 is attached to the forward end portion thereof.

In the center portion of the detachment prevention ring 64, a hole slightly larger than the outer diameter of the sheath 16 is formed. The detachment prevention ring 64 retains the sheath 16 movably in the axial direction.

The sheath manipulating handle 54 has a cylindrical case 68, a support block 70, and a sheath retaining ring 72.

The support block 70 is arranged at the rear end of the sheath manipulating handle 54, and slidably supports the positioning pipe 56 inserted into the sheath manipulating handle 54. Further, as illustrated in FIG. 4B, the forward end side surface of the support block 70 abuts the detachment prevention ring 64 attached to the forward end of the positioning pipe 56, preventing the positioning pipe 56 from being detached from the sheath manipulating handle 54.

The sheath retaining ring 72 is provided at the forward end of the case 68 on the axis of the sheath manipulating handle 54, and fixedly retains the outer periphery of the sheath 16 inserted into the sheath manipulating handle 54. Thus, when the sheath manipulating handle 54 moves, the sheath 16 also moves together with the sheath manipulating handle 54.

The sheath manipulating handle 54 further includes a button 74 protruding out of the case 68 and a claw 76 provided inside the case 68 and interlocked with the movement of the button 74. The claw 76 is urged so as to be pressed against the positioning pipe 56, and is caught by the notches 66 of the positioning pipe 56, thereby performing positioning on the sheath manipulating handle 54 with respect to the wire manipulating handle 52 and stopping the movement thereof.

When the button 74 is depressed, the claw 76 is raised and detached from the notches 66, enabling the wire manipulating handle 52 to move with respect to the sheath manipulating handle 54. When the hand is released from the button 74 and the sheath manipulating handle 54 is moved with respect to the wire manipulating handle 52, its movement is stopped when the claw 76 is caught by the next notch 66. Thus, assuming that the interval between the adjacent notches 66 is one stroke, the sheath manipulating handle 54 and the sheath 16 can move by the stroke length.

When the sheath 16 moves in the axial direction with the movement of the sheath manipulating handle 54, the proximal end portion of the sheath 16 advances through the hole of the detachment prevention ring 64 to enter the interior of the positioning pipe 56.

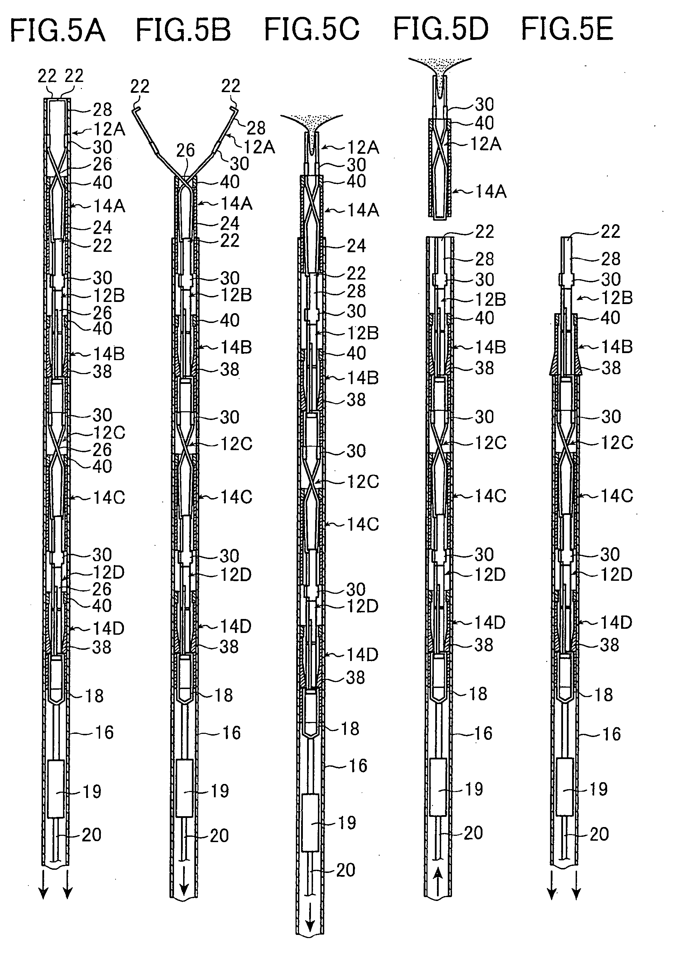

Next, the operation of the clipping device 10 during clipping is described with reference to FIGS. 5A through 5E.

First, as illustrated in FIG. 5A, after four bleeding stop clip units (hereinafter simply referred to as clipping units) formed of the clips 12A through 12D and the connection rings 14A through 14D have been loaded into the sheath 16, the sheath 16 is inserted into the forceps channel of an endoscope. A loading operation of the clipping units can be made, for example, by connecting the dummy clip 18 with the four clipping units, attaching the dummy clip 18 to the forward end of the manipulating wire 20 protruding from the forward end of the sheath 16, moving the sheath 16 forwardly relating to the manipulating wire 20 to make the foremost clip 12A perfectly accommodated within the sheath 16. This loading method will be later described in detail.

As illustrated in FIG. 5A, the forward end of the clip 12A is substantially matched with the forward end of the sheath 16.

The foremost clip 12A is retained in the closed state by the inner wall of the sheath 16. Each of the connection rings 14A through 14D is fitted such that the clamping portion 40 thereof is situated at the initial position, i.e., in the vicinity of the crossing portion 26 of each of the clips 12A through 12D. At this time, the upper ends of the projections 30 of the clips 12B through 12D are respectively situated directly below the connection rings 14A through 14C.

When the forward end of the sheath 16 reaches the forward end of the insert portion of the endoscope inserted into the living body, and protrudes from the forward end of the endoscope, in the manipulating portion 50 illustrated in FIG. 4B, the sheath manipulating handle 54 is pulled such that the claw 76 of the sheath manipulating handle 54 moves from the first notch 66a to the second notch 66b. Because the sheath 16 is fixed to the sheath manipulating handle 54, the sheath 16 retreats by the same amount as the movement amount L of the sheath manipulating handle 54. Through this manipulation, solely the sheath 16 is pulled to the manipulating portion side, with the manipulating wire 20 remaining stationary.

When the sheath 16 is pulled by a predetermined distance corresponding to the interval between the first notch 66a and the second notch 66b, the skirt portions 38 of the foremost connection ring 14A protrude from the forward end of the sheath 16 so that the skirt portions 38 of the connection ring 14A are opened outwards and the claw portions 22, 22 of the clip 12A protruding from the sheath 16 spread due to the urging force to be placed in a state illustrated in FIG. 5B. This places the foremost clip 12A in a usable state. In FIG. 5B, because the skirt portions 38 of the connection ring 14A are placed in a direction perpendicular to the pulling surface, the skirt portions 38 do not appear in the figure.

The connecting portion between the clip 12A and the clip 12B is situated directly below the skirt portions 38 of the connection ring 14A, and hence, in the state as illustrated in FIG. 5B, the forward end of the clip 12B is substantially matched with the forward end of the sheath 16.

When the sheath 16 is pulled, there is exerted a frictional force between the sheath 16 and the connection rings 14A through 14D fitted into the sheath 16. However, between the connection rings 14A through 14D and the clips 12A through 12D, there are exerted the pressurizing force of the clips 12 due to the inner side portions of the closed skirt portions 38, and the pressurizing force applied to the inner wall surfaces of the connection rings 14 (second regions 34 thereof, see FIG. 3B) due to the resilient force of the claw portions 22 of the succeeding clips 12 inclined to open. Further, the projections 30 of the clips 12B through 12D abut the proximal ends of the connection rings 14A through 14C, and cannot enter the holes 43 of the connection rings 14 (see FIG. 3B). Thus, even if the sheath 16 is pulled, the connection rings 14A through 14D make no unnecessary movement. Thus, the connection rings 14A through 14D can maintain the state in which they respectively retain the clips 12A through 12D.

Next, the clipping device 10 in the state of FIG. 5B is moved to press the claw portions 22, 22 of the diverged clip 12A against the portion to be subjected to clipping, and the lever 60 of the manipulating portion 50 (see FIGS. 4A and 4B) is pulled, whereby the manipulating wire 20 is pulled by a predetermined amount. By pulling the manipulating wire 20, the clips 12A through 12D engaged sequentially starting from the dummy clip 18 are pulled all together.

At this time, in the states illustrated in FIGS. 5B and 5C, the skirt portions 38 of the connection ring 14A protruding from the forward end of the sheath 16 are opened, and the retention of the clip 12A by the pressure of the skirt portions 38 is cancelled. Further, because the skirt portions 38 are opened at the forward end of the sheath 16, the connection ring 14A is prevented from retreating into the sheath 16. Therefore, as illustrated in FIG. 5C, the foremost clip 12A retreats with respect to the connection ring 14A, when the manipulating wire 20 is pulled. The lower ends of the projections 30 of the clip 12A are pushed in the forward end of the connection ring 14A, i.e., the clamping portion 40, whereby clamping of the clip 12A by the connection ring 14A is completed.

The engaged portion between the clip 12A and the next clip 12B comes off from the rear end of the connection ring 14A at the same time as the clamping of the clip 12A by the connection ring 14A. When the engaged portion between the clip 12A and the clip 12B comes off from the connection ring 14A, the arm portions 28 of the clip 12B are diverged until they abut the inner wall of the sheath 16 due to a resilient force, and the claw portions 22, 22 of the clip 12B are diverged more widely than the width of the turned portion 24 of the clip 12A, whereby the connection between the clip 12A and the clip 12B is cancelled. This enables the clip 12A and the connection ring 14A to be detached from the sheath 16, whereby the clipping by the clip 12A and the connection ring 14A is completed.

On the other hand, the succeeding clips 12B through 12D are retained by the connection rings 14B through 14D whose skirt portions 38 are closed so as not to move in the rotating direction and the advancing/retreating direction with respect to the connection rings 14B through 14D. Further, the claw portions 22 are pressed against the inner walls of the second regions 34 (see FIG. 3B) of the connection rings 14B through 14D by the expanding force (urging force) of the claw portions 22 of the clips 12C through 12D engaged with the clips 12B through 12D and the claw portions of the dummy clip 18, with the result that the frictional force between the clips 12B through 12D and the connection rings 14B through 14D is enhanced. Thus, the connection rings 14B through 14D move with the movement of the clips 12B trough 12D.

That is, the clips and the connection rings other than the foremost clip 12A and the connection ring 14A retaining the same, i.e., the clips 12B through 12D and the connection rings 14B through 14D advance and retreat integrally with respect to the sheath 16, and the connected state of the clips 14B through 14D and the dummy clip 18 is maintained by the connection rings 14B through 14D.

The manipulating wire 20 is constructed so as to be capable of being pulled by a fixed amount from the initial state. This fixed amount is an amount equal to the length of the second regions 34 of the connection rings 14 or an amount slightly larger than that, and at the same time, it is an amount equal to the length from the lower ends of the projections 30 of each clip 12 to the forward end of the connection ring 14 retaining that clip 12, or an amount slightly smaller than that. In the manipulating portion 50 of FIG. 4A, this fixed amount is determined by the length as measured from the home position of the lever 60 to the movement limit at the rear.

After it has been pulled by the fixed amount, the manipulating wire 20 is soon restored by that fixed amount due to the spring 62 urging the lever 60 of the manipulating portion 50. When the pulling force of the lever 60 is canceled at the manipulating portion 50, the lever 60 is restored to the former position, and the manipulating wire 20 pulled from the state illustrated in FIG. 5B to the state illustrated in FIG. 5C is thereby restored to the former position, whereby the state as illustrated in FIG. 5D is attained. That is, as in the case of FIG. 5B, the forward end of the second clip 12B is restored to the position where it is substantially matched with the forward end of the sheath 16.

Next, by moving the claw 76 from the second notch 66b to the third notch 66c in the manipulating portion 50 in FIG. 4A in order to place the second clip 12B in a usable state, the sheath 16 is pulled to the manipulating portion side by predetermined one stroke, i.e., by a predetermined interval.

The predetermined interval refers to the distance between the forward ends of adjacent two clips 12 loaded in the sheath 16, i.e., the load interval of the clips 12 in the sheath 16.

The predetermined interval corresponds to the length from the second notch 66b to the third notch 66c, i.e., the length of one stroke by which the sheath 16 is pulled. Therefore, when the claw 76 is moved from the second notch 66b to the third notch 66c, the sheath 16 moves to the manipulating portion side by the predetermined interval L, and the clip 12B shifts from the state in which the position of the forward end is substantially matched with the position of the forward end of the sheath 16 as illustrated in FIG. 5D to the state in which the clipping can be performed as illustrated in FIG. 5E.

After that, the manipulating wire 20 is pulled by a predetermined amount with the claw portions of the clip 12B being pressed against a site desired to be subjected to clipping in the same way as in the clip 12A described above. This cancels the connection between the clip 12B and the clip 12C at the same time as the completion of the clamping of the clip 12B by the connection ring 14B, and the clipping by the clip 12B is completed.

Regarding the clips 12C and 12D, the clipping is performed in the same way as in the clip 12B.

Next, a clip package according to the invention will be described.

The clip package according to the invention described below is a connected clip package encasing a given number of connected clip units (each being the clip 12 fitted with the connection ring 14) used in the successive clipping device 10 described above. The clip package of the invention may of course be a single piece of clip unit used in a single clipping device (a single clip 12 and a single dummy clip 18 fitted with a single connection ring 14).

### FIRST EMBODIMENT:

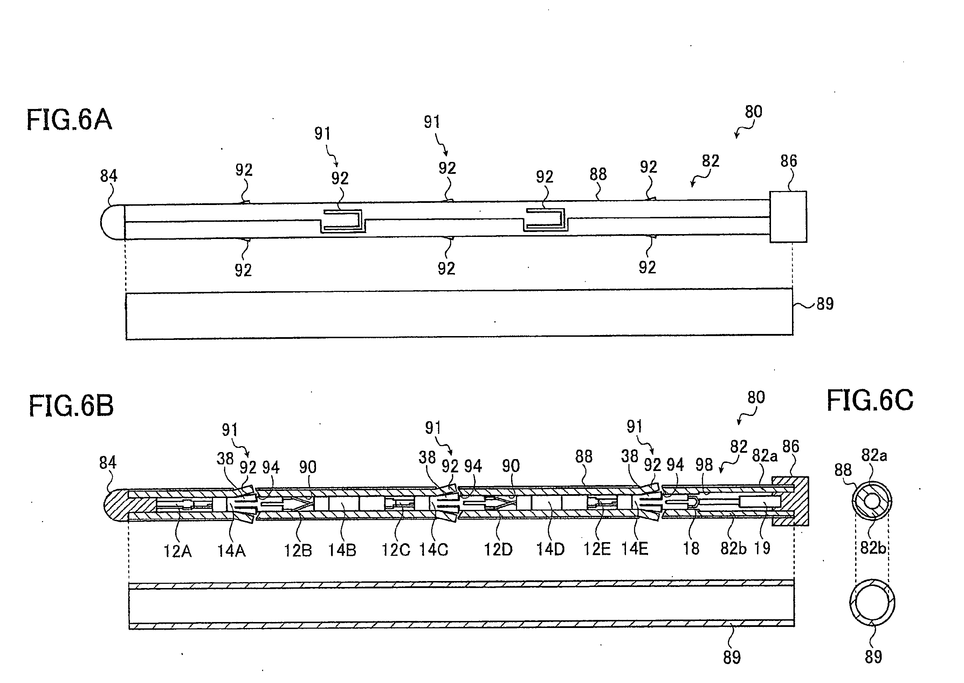

FIGS. 6A through 6C are a side view, a side sectional view, and a front sectional view, respectively, illustrating a connected clip package according to a first embodiment. In the following description, the left end is referred to as a forward end and the right end is referred to as a proximal end in FIGS. 6A and 6B.

The above description of the clipping device 10 has been made referring to an example comprising four connected clip units. The description below will be made by way of an example comprising five connected clip units.

As illustrated in FIG. 6A, a successive clip package 80 comprises a case 82, an upper cap 84, a lower cap 86, and an outer tube 89.

The case 82 has a cylindrical shape and contains clip units each comprising a clip 12 and a connection ring 14. As illustrated in FIGS. 6A and 6C, the case 82 is formed of two case parts (upper and lower halves) 82a and 82b each substantially semi-cylindrical and symmetrical with respect to the axis of the cylinder. The two case parts 82a and 82b are fitted with the upper cap 84 on the forward ends and the lower cap 86 on the proximal ends to keep the case 82 closed.

The case part 82a and the case part 82b have a border that detours openings 91 described later. More specifically, the case part 82a and the case part 82b each have a shape such that their border changes its circumferential position so that each opening 91 is not located over both case parts but only formed in either the case part 82a or the case part 82b.

Preferably, the case 82 is transparent or translucent so that the inside is visible. Further, the case 82 is preferably formed of a resin that does not change in quality in an ambient temperature range (e.g., 5°C to 38°C) for impact resistance and ease of use, and for ease with which it can be shaped into a desired form. Although the case 82 has a cylindrical shape according to this example, its external shape is not limited to a cylinder and may be a prism.

The case 82 has a transparent plastic cover 88 covering the outer peripheries of the case parts 82a and 82b. The regions corresponding to openings 91 described later are not covered with the cover 88. Covering the outer peripheries of the case parts 82a and 82b with the cover 88 keeps the case parts 82a and 82b from splitting from each other. The case 82 may have the case parts 82a and 82b bonded to each other with an adhesive or other means.

The upper cap 84 and the lower cap 86 may be formed of any material such as rubber and resin, provided that they can close the forward ends and the proximal ends of the case parts 82a and 82b. The lower cap 86 is removable. When loading the sheath with the clip units inside the case 82, the lower cap 86 is removed, and the clip units inside, left connected, are withdrawn. The upper cap 84 may or may not be removable. Further, the case 82 may be adapted to form also the forward end, dispensing with the upper cap 84.

As illustrated in FIG. 6B, the case 82 has a through-hole formed throughout the length of the case 82. The through-hole has a diameter slightly greater than the outer diameter of the connection rings 14 and substantially equal to the inner diameter of the sheath 16 in which the clip units are to be loaded. Located in the through-hole are five clips 12A to 12E, a dummy clip 18, and five connection rings 14A to 14E covering the joints. The forward end of the foremost clip 12A is protected by a projection projecting from the upper cap 84 inwardly into the case 82. The lower cap 86 holds the connecting member 19 located at the proximal end of the dummy clip 18 connected to the rearmost clip 12E.

A sheath recess 98 is formed near the proximal end of the case 82 to receive and engage the sheath 16. The sheath recess 98 has substantially the same diameter as the outer diameter of the sheath 16 into which the clips 12A to 12E and the connection rings 14A to 14E are to be loaded. There is formed on the forward end of the sheath recess 98 a step corresponding to the difference between the inner diameter of a straight section 90 of the case 82 and the inner diameter of the sheath recess 98, the difference being approximately equivalent to the thickness of the sheath 16, When loading the sheath 16 with the clip units located inside the case 82, the sheath 16 is inserted up to the forward end of the sheath recess 98.

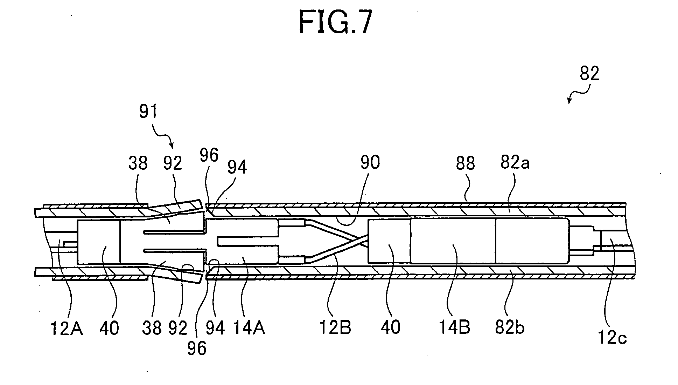

FIG. 7 is a view illustrating a part of FIG. 6B enlarged. The case 82 has openings 91 provided at positions where the connection rings 14A to 14E are located for the skirt portions 38. The openings 91 are each formed of a flap 92.

Each flap 92 is a plate member having a forward end side connected to the straight section 90 and the other three sides cut out from the straight section 90. Each flap 92 covers a region opposite the corresponding skirt portion 38. The flaps 92 have substantially the same thickness as the straight section 90.

Each flap 92 is pivotable about the axis at its joint with the adjacent straight section 90 in the direction away from the skirt portion 38. When free from any external force, each flap 92 radially and outwardly extends at substantially the same angle as the skirt portion in a natural state and over substantially the same range as the skirt portion 38.

Although each flap 92 and the straight section 90 are integrally formed in this embodiment, the invention is not limited to such a configuration. Each flap 92 and the straight section 90 may be formed of different pieces.

Each flap 92 need only hold the adjacent skirt portion 38 in a natural state with no external force applied. Accordingly, each flap 92 need not be limited to the shape such that, when free from any external force, each flap 92 radially extends outwardly at substantially the same angle as the skirt portion in a natural state and over substantially the same range as the skirt portion 38. When no external force is applied, each flap 92 may be in the same plane as the straight section 90. In this case, each flap 92 may be formed to have only a low resistance in the pivotal direction to permit easy pivoting so as to radially expand outwardly as it is pressed outwardly by the expanding force of the adjacent skirt portion 38.

Further, the case 82 is formed with a slant portion 94 at a surface thereof opposite each of the proximal end side of the flap 92. Each slant portion 94 has an end thereof closer to the adjacent flap 92 wider in the radial direction than the straight section, decreasing in size in the radial direction toward the proximal end.

As described above, the clips 12A to 12E are connected to each other such that one is twisted 90° with respect to the adjacent ones. The connection rings 14A to 14E are fitted to the clips 12A to 12E such that the connection rings are twisted 90° with respect to the preceding and following connection rings 14. Accordingly, the openings 91 formed in the case 82 at the positions corresponding to the connection rings 14A through 14E are also twisted 90° with respect to each other. In FIG. 6B, the openings 91 corresponding to the skirt portions 38 of each of the connection rings 14A, 14C and 14E are shown in the two upper and lower positions. The openings 91 corresponding to the skirt portions 38 of each of the connection rings 14B and 14D are formed in two positions in the direction vertical to FIG. 6B.

The flaps 92 provided at the openings 91 allow the connection rings 14A to 14E to be housed within the case 82 in an expanding state without any external force being applied to the skirt portions 38. This prevents the elasticity of the skirt portions 38 from deteriorating during the storage in the case 82 and hence maintains the performance of the connection rings 14A to 14E.

As illustrated in FIGS. 6A through 6C, the outer tube 89 is a cylindrical tube having an inner diameter substantially equal to the outer diameter of the case 82 and freely movably placed over the outer periphery of the case 82. At least the inner periphery of the outer tube 89 is formed of a material having a higher rigidity than the case 82. The outer tube 89 allows the case 82 to be freely movably inserted therein.

With the clip package 80 of the invention configured as above, when the clips 12A to 12E and the connection rings 14A to 14E are withdrawn from the case 82, the outer tube 89 is fitted over the outer periphery of the case 82 so that the inner periphery of the outer tube 89 keeps the flaps 92 at the openings 91 depressed toward the skirt portions 38 to close the skirt portions 38. Further, each slant portion 94 provided on the side of the adjacent opening 91 closer to the proximal end serves to prevent the skirt portion 38 from being turned over as the skirt portion 38 leaves the opening 91.

Thus, the inner periphery of the outer tube 89 formed of a material having a higher rigidity than the case 82 forms a skirt retracting mechanism in the present invention.

The clip units are housed into the case 82 as follows.

First, the clips 12A to 12E are connected in order. The clips 12A to 12E are connected in such a manner that the slits 46 of each connection ring 14 fitted onto a clip 12 are widened, the turned portion 24 of one clip 12 is engaged with the claw portions 22 of the next clip 12, and the joint is positioned at a given location in each connection ring 14. The last clip 12E is fitted with the dummy clip 18 in the same manner.

An assembly of previously connected clips is placed in the case part 82a of the case 82. Then, the other case part 82b is placed on the case part 82a, their outer peripheries are covered with the cover 88, and the upper cap 84 and the lower cap 86 are fitted to obtain a connected clip package 80.

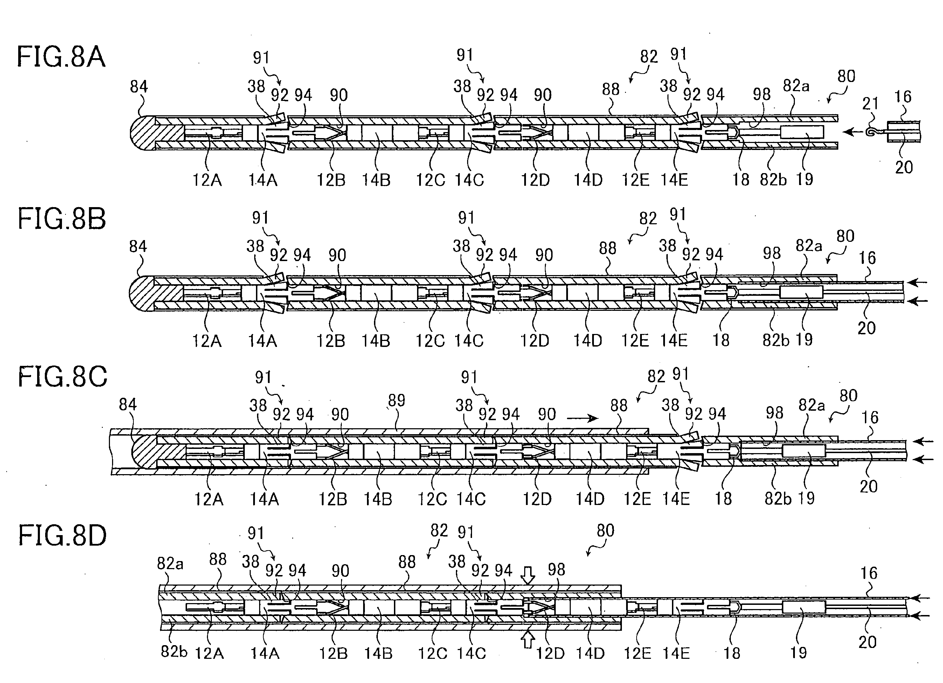

Next, a description will be made referring to FIGS. 8A to 8D on how the clip units are withdrawn from the connected clip package 80 and loaded into the sheath 10.

First, as illustrated in FIG. 8A, the lower cap 86 of the connected clip package 80 is removed, and the connecting member 21 at the end of the manipulating wire 20 projecting from the sheath 16 is connected to the connecting member 19 at the proximal end of the dummy clip 18 inside the case 82.

The manipulating wire 20 can be withdrawn from the sheath 16 using a manipulating portion 50 illustrated in FIGS. 4A to 4B. Specifically, after all the clips 12 in the clipping device 10 illustrated in FIGS. 1A and 1B have been used, the sheath manipulating handle 54 of the manipulating portion 50 is located closer to the wire manipulating handle 52. For example, the claw 76 of the sheath manipulating handle 54 is engaged in the rearmost notch 66. In that state, the wire manipulating handle 52 and the sheath manipulating handle 54 are separated by a given distance. The manipulating wire 20 can be withdrawn from the forward end of the sheath 16 by pulling the sheath manipulating handle 54 by that distance to cause the sheath 16 to be pulled with respect to the manipulating wire 20. A positioning pipe 56 may be provided with notches at intervals corresponding to the distance by which the manipulating wire 20 is withdrawn.

The dummy clip 18 engaged with the used, rearmost the clip 12 is removed in advance, with the manipulating wire 20 projecting from the sheath 16.

The connecting member 19 and the connecting member 21 are removable but do not disengage in the forward and backward movements of the manipulating wire 20. For example, the connecting member 19 has a connecting ring, and the manipulating wire 20 has the connecting member 21 in the form of a hook. The connecting member 21 in the form of a hook of the manipulating wire 20 is engaged with the connecting ring of the connecting member 19 to link the dummy clip 18 and the manipulating wire 20.

Upon connecting the manipulating wire 20 to the dummy clip 18 inside the case 82, the sheath 16 is inserted up to the forward end of the sheath recess 98 and engaged with the case 82 as illustrated in FIG. 8B. The operator, holding the sheath 16 and the case 82, inserts the end of the sheath 16 into the sheath recess 98 of the case 82 and pushes the sheath manipulating handle 54 of the manipulating portion 50 forward with respect to the wire manipulating handle 52 to advance the sheath 16 relative to the manipulating wire 20, accomplishing insertion of the sheath 16 up to the forward end of the sheath recess 98.

With the sheath 16 engaged in the sheath recess 98 of the case 82, the inner diameter of the straight section 90 of the case 82 is substantially equal to the outer diameter of the sheath 16.

Then, as illustrated in FIG. 8C, the outer tube 89 is fitted over the case 82 from the forward end of the case 82 to cover the outer periphery of the case 82 with the outer tube 89. In other words, the case 82 is inserted into the outer tube 89 from its forward end.

The outer tube 89 placed over the outer periphery of the case 82 depresses the flaps 92, which are bent outwards from the outer periphery of the case 82 corresponding to the straight section 90, toward the skirt portions 38. The flaps 92, depressed by the outer tube 89 toward the skirt portions 38, have the surface thereof facing the skirt portions moved into a plane identical to that of the straight section 90. Thus, an external force acting toward the axis of the case 82 is applied also onto the skirt portion 38 and causes the skirt portion 38 to pivot toward the axis of the case 82, retracting the skirt portion 38 inside the connection ring 14A.

As illustrated in FIG. 8D, only the sheath 16 is moved toward the forward end along with the case 82, with the manipulating wire 20 kept in position. The displacement of the sheath 16 is achieved by moving the sheath manipulating handle 54 of the manipulating portion 50 toward the forward end relative to the wire manipulating handle 52. Preferably, the sheath 16 and the case 82 are moved while holding region of the case 82 near the forward end of the sheath recess 98 indicated by arrows in FIG. 8D. The displacement of the sheath 16 and the case 82 causes the clips 12A to 12E and the connection rings 14A to 14E inside the case 82 are inserted in sequence from the rearmost end into the sheath 16.

The clips 12 may be loaded into the sheath 16 by moving the sheath 16 forward relative to the manipulating wire 20 until the foremost first clip 12A is fully retracted into the sheath 16. The clips 12 may alternatively be loaded into the sheath 16 by pulling the manipulating wire 20 to draw the clip units into the sheath 16 instead of moving the sheath 16 and the case 82.

The skirt portions 38 of the connection rings 14A to 14E are open in the case 82 where they are located in the positions opposite the openings 91. When the case 82 moves toward the forward end, the skirt portions 38 are kept inside the connection rings 14 and by the flaps 91 that are in turn depressed by the outer tube 89 so that the skirt portions 38 are contained within the straight section 90 and thus can be withdrawn into the sheath 16. The skirt portions 38 of the connection rings 14A to 14E pass the other openings 91 located closer to the proximal end. The surface of the flaps 92 in contact with the skirt portions 38 is in the same plane as the straight section 90 so that the skirt portions 38 can pass the openings 91 without being turned over.

The connection rings 14A to 14E have the inner walls of the second region 34 (see FIG. 3B) on the proximal side urged by a force of the claws 22 of the following clips 12B to 12E and the dummy clip 18 tending to expand. Accordingly, the connection between the clips 12 and the positional relationships between the clips 12A to 12E and the connection rings 14A to 14E inside the case 82 can be maintained.

Further, when loading the sheath 16 with the clips, the clips 12A to 12E in the connection rings 14A to 14E are urged by the inner wall of the skirt portions 38, which are closed, and thus the connection rings 14A to 14E maintain the connection between the clips 12A to 12E and the dummy clip 18. This prevents disengagement among the clips 12A to 12E and the dummy clip 18 and their displacement relative to the connection rings 14A to 14E when loading the sheath 16 with the clips.

Loading of the clips 12 into the sheath 16 is completed when the forward end of the sheath 16 has been moved to a position where the forward end of the foremost clip 12A is contained in the forward end of the sheath 16. When loading is completed, the manipulating portion 50 has its sheath manipulating handle 54 moved forward, with the claw 76 hooked in the first notch 66.

Thus, the connected clip package 80 can be distributed and stored as an assembly of connected clips 12. Further, the clips can be easily loaded into the sheath 16 with their connections being kept. This greatly reduces operator burden and permits quick and easy.loading of clip units or a series of clips consisting of clips 12.

Further, since the connected clip package 80 can be stored with the skirt portions 38 of the connection rings 14 allowed to be open, deterioration of the elasticity of the skirt portions 38 can be prevented, and the clips 12 and the connection rings 14 can exercise their full performances when the clips 12 are used (in the clipping operations).

Further, the connected clip package 80 permits easy loading operation because of its configuration such that the skirt portions 38, left open, are automatically closed simply by covering the outer periphery of the case 82 with the outer tube 89 when loading the clips into the sheath 16.

Further, since the skirt portions can be closed by applying an external transverse force onto them in their pivotal direction in lieu of exerting a load onto them in a direction opposite that in which they move, no unnecessary load is applied to the skirt portions, and hence breakage of the skirt portions in the loading process can be prevented.

### SECOND EMBODIMENT:

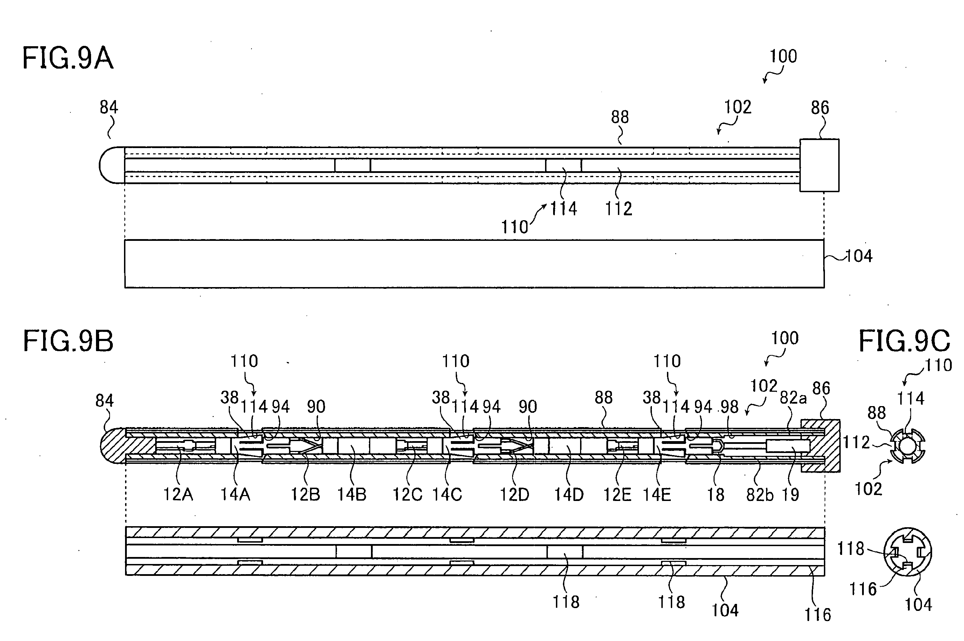

FIGS. 9A through 9C are a side view, a side sectional view, and a front sectional view, respectively, illustrating a connected clip package 100 according to a second embodiment.

The connected clip package 100 comprises a case 102, an outer tube 104, and an upper cap 84 and a lower cap 86.

The connected clip package 100 has guide grooves 112 formed in the outer periphery of the case 102 extending along the axis of the cylinder and passing through openings 110, thus forming apertures 114 at the openings 110. The outer tube 104 comprises guide ridges 116 and the projections 118. The guide ridges 116 are formed on the inner periphery of the outer tube 104 in positions corresponding to the guide grooves 112 and extend along the axis of the cylinder so as to engage with the guide grooves 112. The projections 118 are formed on the guide ridges 116 in positions corresponding to the openings 110 to engage with the apertures 114. The case 102 and the outer tube 104 otherwise have basically the same configurations as the case 82 and the outer tube 89 described above. The same components will therefore not be described in detail below.

The upper cap 84 fitted on the forward end and the lower cap 86 fitted on the proximal end of the case 102 are the same as the upper cap 84 and the lower cap 86 of the connected clip package 80 described above.

The case 102 is a cylindrical member formed with the guide grooves 112 and the apertures 114 forming the openings 110.

The guide grooves 112 are grooves formed in the outer periphery of the case 102 extending along the axis of the cylinder and passing through regions 110 corresponding to the openings 110.

The apertures 114 each provide a space forming the opening 110 and are located in the regions corresponding to the skirt portions 38. The skirt portions 38 of the connection rings 14 of the series of clips disposed inside the case 102 partially project into the apertures 114. With the apertures 114 formed in the regions of the case 102 corresponding to the skirt portions 38, the skirt portions 38 can be held inside the case 102 in a natural state free from any external force.

The outer tube 104 is a cylindrical member having substantially the same inner diameter as the outer diameter of the case 102 and comprises on the inner periphery thereof guide ridges 116 and the projections 118, which act as skirt retracting mechanism in the present invention.

The guide ridges 116 are linear ridges formed in the regions of the outer periphery corresponding to the guide grooves 112 and extend along the axis of the cylinder. The guide ridges 116 engage with the guide grooves 112 as the case 102 is inserted into the outer tube 104.

The projections 118 are provided on the guide ridges 116 in positions corresponding to the openings 110. The distance from the center axis of the outer tube 104 to the top surface of the projections 118 is equal to the distance from the center axis of the case 102 to the inner surface of the straight section.

The projections 118 engage with the apertures 114 as they come to be located opposite the apertures when the case 102 is inserted into the outer tube 104.

The clip package 100 has the above configuration.

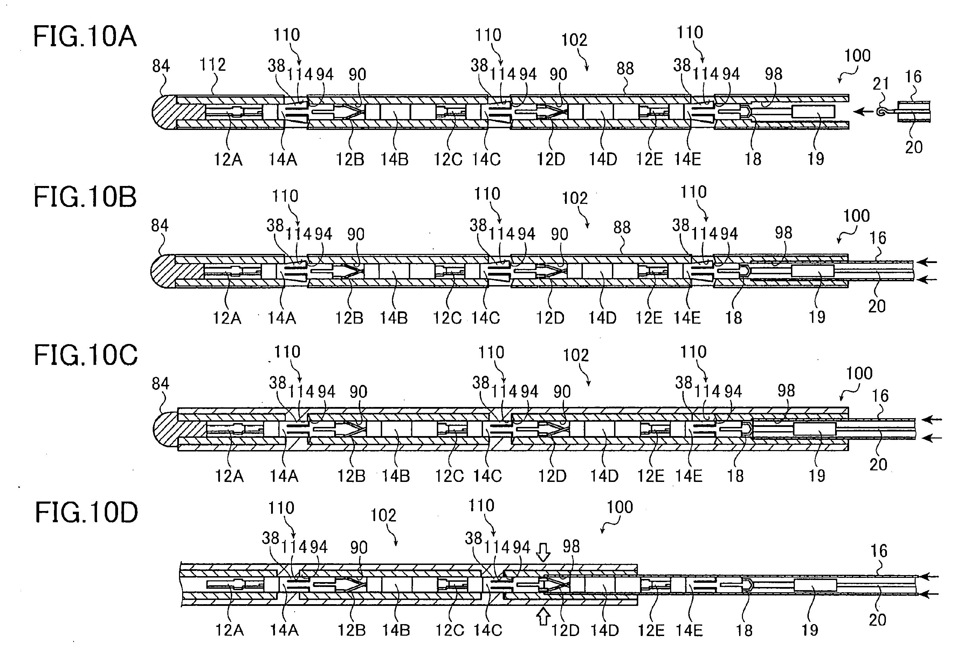

Next, a description will be made on how the clip units are loaded from the connected clip package 100 into the sheath 16 referring to FIGS. 10A to 10D. The method of fabricating the connected clip package 100 is the same as for the clip package 80 and will not be described below.

First, as illustrated in FIG. 10A, the lower cap 86 of the connected clip package 100 is removed, and the manipulating wire 20 caused to project from the sheath 16 is connected to the connecting member 19 at the proximal end of the dummy clip 18 inside the case 102.

Upon connecting the manipulating wire 20 to the dummy clip 18 inside the case 102, the sheath 16 is inserted up to the forward end of the sheath recess 98 and engaged with the case 102 as illustrated in FIG. 10B.

Then, as illustrated in FIG. 10C, the outer tube 104 is fitted over the case 102 from the forward end of the case 102 to cover the outer periphery of the case 102 with the outer tube 104. The operator inserts the case 102 into the outer tube 104 in such a radial direction that the guide grooves 112 of the case 102 engage with the guide ridges 116 of the outer tube 104 and the projections 118 engage in the apertures 114.

With the projections 118 engaged in the apertures 114, the surface of the projections 118 facing the skirt portions 38 is positioned in the same plane as the inner wall of the straight section 90. Thus, the projections 118 exert an external force acting toward the axis onto the skirt portion 38, and cause the skirt portion 38 to pivot toward the axis, retracting the skirt portion 38 inside the connection ring 14A.

As illustrated in FIG. 10D, only the sheath 16 is moved toward the forward end along with the case 102, with the manipulating wire 20 kept in position. The displacement of the sheath 16 and the case 102 causes the clips 12A to 12E and the connection rings 14A to 14E in the case 102 to be loaded in sequence from the rearmost end thereof into the sheath 16.

Thus, the skirt portions can be allowed to assume their natural posture when in storage and distribution and closed when loading the clips, and this is achieved also by the above configuration such that the openings have apertures and the case has no member to come into contact with the skirt portions whereas the outer tube has projections to engage in the apertures, as illustrated in the clip package 100.

Thus, the openings need not be limited to the configuration having the flaps such that the outer tube exerts an external force onto the skirt portions through parts of the case; the openings may have apertures such that the outer tube come into direct contact with the skirt portions and the outer tube exerts an external force onto the skirt portions.

In addition, as illustrated in the clip package 100, the grooves provided in the outer periphery of the case and the ridges provided on the inner periphery of the outer tube prevent circumferential displacement relative to each other and ensure firm engagement of the apertures and the projections.

Further, the grooves formed in the case and the ridges provided in the outer tube allow the apertures to be moderately deep and the ridges to be moderately high, which makes it easier to insert the case into the outer tube.

In this embodiment, since the circumferential position of the skirt portions differs from joint to joint, grooves for determining their angles may be provided in regions other than those corresponding to the apertures.

### THIRD EMBODIMENT:

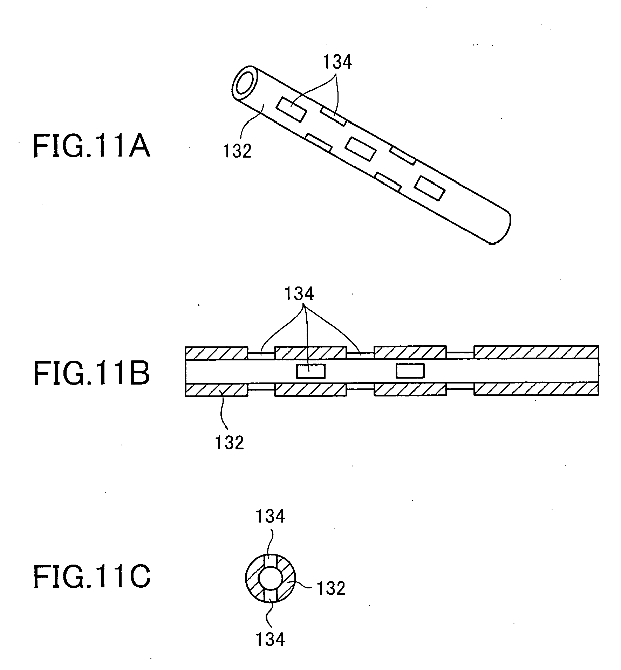

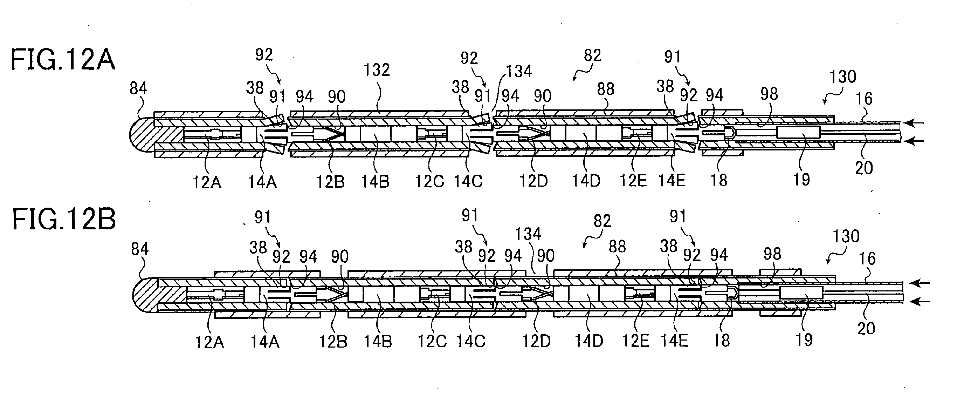

FIGS. 11A through 11C are a perspective view, a side sectional view, and a front sectional view, respectively, illustrating an outer tube 132 used for a connected clip package 130 according to a third embodiment. FIGS. 12A through 12B are cross sectional views illustrating the connected clip package 130.

The case used for the connected clip package 130 is the same as the case 82 illustrated in FIGS. 6A to 6C, and views and therefore descriptions thereof will not be given.

The outer tube 132 is a cylindrical member having substantially the same inner diameter as the outer diameter of the case and comprises apertures 134 formed in the regions corresponding to the openings 91.

As illustrated in FIGS. 12A and 12B, the connected clip package 130 has the case 82 housed inside the outer tube 132, that is to say, the outer periphery of the case 82 is covered with the outer tube 132, already from when it is in storage and distribution.

In storage and distribution, the connected clip package 130 has the case 82 and the outer tube 132 disposed in a relative position such that the apertures 134 of the outer tube 132 are located opposite the openings 91 of the case 82 as illustrated in FIG. 12A.

When the apertures 134 and the openings 91 are opposite to each other, the flaps 92 of the openings 91 are free from external force and thus the skirt portions 38 are allowed to assume their natural posture.

Next, when loading the clips located in the case 82 into the sheath 16, the outer tube 132 is relative to the case 82 moved toward the proximal end of the case 82 along the axis of the case 82 as illustrated in FIG. 12B. The distance of the relative movement is longer than the length of the aperture 134 along the axis of the case 82.

Moving the outer tube 132 relative to the case 82 toward the proximal end thereof locates the openings 91 of the case 82 opposite other regions than the apertures 134 of the outer tube 132 so that the outer periphery of the openings 91 is closed by the inner periphery of the outer tube 132. Thus, the flaps 92 of the openings 91 are moved toward the axis of the case 82 by an external force exerted by the outer tube 132 and acting toward the axis of the case 82. Movement of the flaps 92 toward the axis of the case 82 causes external force to be applied onto the skirt portions 38, closing the skirt portions 38.

Thus, according to the configuration wherein the apertures are formed in the outer tube for the openings in the case, the skirt portions are allowed to assume their natural posture also when the case is housed in the outer tube. Accordingly, the case can be kept in the outer tube already from when it is storage and distribution, and the operator is therefore saved the trouble of inserting the case into the outer tube and need only move the case housed in the outer tube relative to the outer tube to close the skirt portions.

### FOURTH EMBODIMENT:

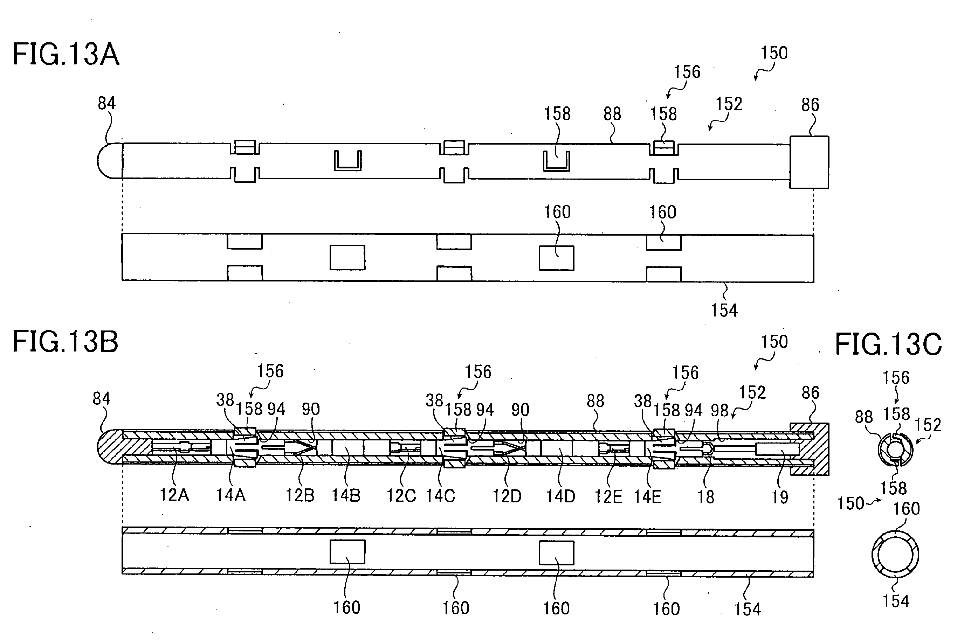

FIGS. 13A through 13C are a side view, a side sectional view, and a front sectional view, respectively, illustrating a successive clip package 150 according to a fourth embodiment.

The connected clip package 150 comprises a case 152, an outer tube 154, and the upper cap 84 and the lower cap 86.

The connected clip package 150 basically has the same configurations as the case 82 and the outer tube 89 described above except that the former has apertures 160 formed in the outer tube 154. The same components, therefore, will not be described in detail below.

The upper cap 84 fitted on the forward end and the lower cap 86 fitted on the proximal end of the case 152 are the same as the upper cap 84 and the lower cap 86 of the connected clip package 80 described above.

The case 152 is a cylindrical member comprising the openings 156 in the positions corresponding to the skirt portions 38. The openings 156 each comprise a flap 158.

Each flap 158 is a plate member having an end side in direction of circumference connected to the straight section 90 as shown in FIG. 13C and the other three sides separated from the straight section 90. Each flap 158 covers the region opposite the corresponding skirt portion 38. The flaps 158 at least partially have substantially the same thickness as the straight section 90. In this embodiment, the flaps 158 have a shape such that the thickness increases with the distance from the joint with the straight section 90.

Each flap 158 is also pivotable about the side where it meets with the straight section 90 in the direction away from the skirt section 38. Each flap 158 is open farther outwards than the adjacent skirt portion 38 so that the skirt portion 38 may assume a natural posture when the skirt portion 38 is free from external force.

The outer tube 154 is a cylindrical member having substantially the same inner diameter as the outer diameter of the case and comprises apertures 160 formed in the regions corresponding to the openings 156.

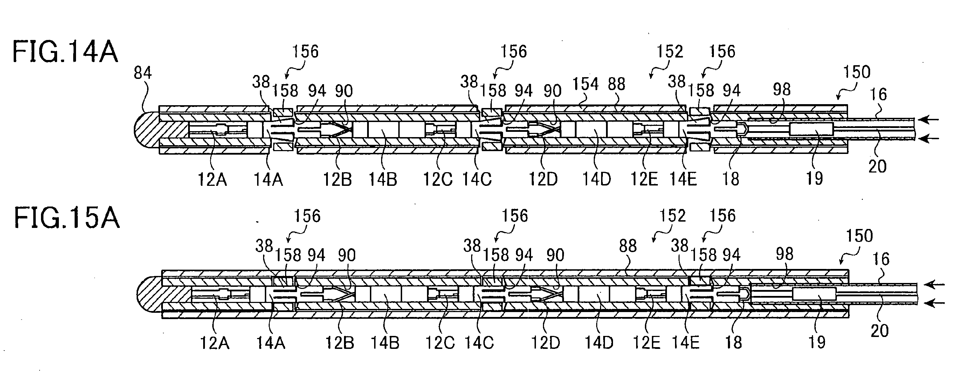

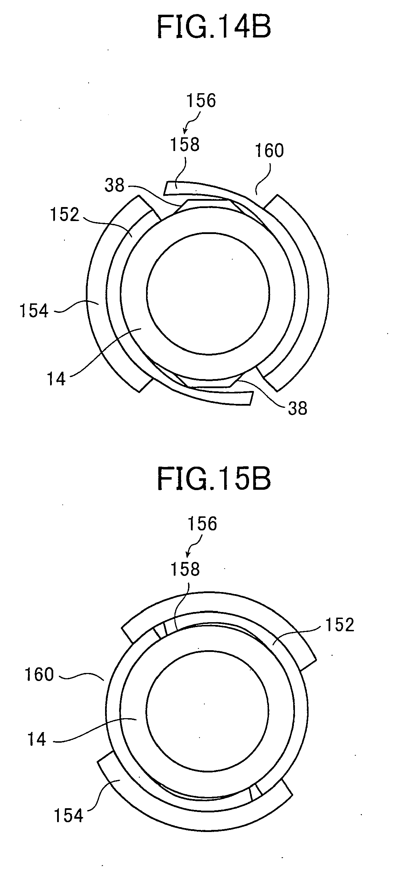

As illustrated in FIGS. 14A and 14B and FIGS. 15A and 15B, the connected clip package 150 also has the case 152 inserted in the outer tube 154, that is to say, the outer periphery of the case 152 is covered with the outer tube 154, already from storage and distribution.

In storage and distribution, the connected clip package 150 has the case 152 and the outer tube 154 disposed in a relative position such that the apertures 160 of the outer tube 154 are located opposite the openings 156 of the case 152 as illustrated in FIGS. 14A and 14B.

When the apertures 160 and the openings 156 are opposite to each other, the flaps 158 of the openings 156 are free from external force and thus the skirt portions 38 are allowed to assume their natural posture.

Next, when loading the clips located inside the case 152 into the sheath 16, the outer tube 154 is turned counterclockwise relative to the case 152 as seen from the proximal end of the case toward the forward end of the case 152 as illustrated in FIGS. 15A and 15B. The distance of the relative movement along the inner periphery of the outer tube 154 in the circumferential direction is longer than the length of the apertures 160 along the case 152 in the circumferential direction.

Turning the outer tube 154 relative to the case 152 locates the openings 156 of the case 152 opposite the other regions than the apertures 160 of the outer tube 154 so that the outer periphery of the openings 156 are closed by the inner periphery of the outer tube 154. Thus, the flaps 158 of the openings 156 are moved toward the axis of the case 152 by an external force exerted by the outer tube 154 and acting toward the axis of the case 152. Movement of the flaps 158 toward the axis of the case 152 causes external force to be applied onto the skirt portions 38, closing the skirt portions 152.

Thus, the skirt portions are allowed to assume their natural posture when in storage and distribution and closed merely by turning the outer tube and the case relative to each other in a given direction when loading the sheath with the clips, and this is achieved also by the configuration such that the flaps each have its end side in the circumferential direction connected with the straight section.

### FIFTH EMBODIMENT:

Preferably, a protuberance is provided on the surface of each flap used as an opening that is in contact with the adjacent skirt portion and/or the surface of each skirt portion in contact with the adjacent flap. Such a protuberance reduces the contact area between the skirt portions and the flaps, which in turn reduces the resistance produced by the relative movement. Thus, the clips and the connection rings can be smoothly moved inside the case.

Further, the protuberance allows the proximal end of each skirt portion to retract farther toward the axis than the plane where the straight section lies, which prevents the skirt portion from being hooked to the case with an increased certainty.

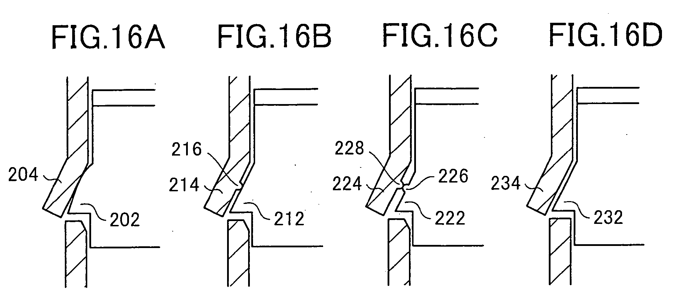

FIGS. 16A to 16D are partial sectional views illustrating relationships between a skirt portion and a flap of the connected clip package in variations of the fifth embodiment.

For example, as illustrated in FIG. 16A, the plane of a skirt portion 202 in contact with a flap 204 (outer surface) may be convex, i.e., a plane curved outwardly toward the flap 204 farther than the plane containing the forward end and the proximal end of the skirt portion 202, a polyhedral plane, etc. In this case, the highest protrusion is the above protuberance.

Thus, the outer surface of the skirt portion 202 having a convex surface reduces its contact area with the flap 204 and moves the proximal end of the skirt portion 202 farther toward the axis when the flap 204 exerts an external force onto the skirt portion 202.

As illustrated in FIG. 16B, the surface of a flap 214 in contact with a skirt portion 212 may be provided with a protuberance 216 protruding toward the skirt portion 212.

Thus, provision of the protuberance 216 also produces the same effects as in the case illustrated in FIG. 16A.

Alternatively, there may be provided a protuberance 226 on the surface of a skirt portion 222 in contact with a flap 224 and a protuberance 228 on the surface of a flap 224 in contact with the skirt portion 222 as illustrated in FIG. 16C.

Thus, provision of such a protuberance on both the skirt portion 222 and the flap 224 reduces the contact area with an increased certainty and moves the proximal end of the skirt portion 202 farther toward the axis with an increased certainty.

The planes of a skirt portion 232 and a flap 234 in contact with each other may be both flat as illustrate in FIG. 16D although this configuration does not produce the effects of reducing the resistance generated in the relative movement and preventing the skirt portion from being hooked to the case with an increased certainty.

In all the above embodiments, the case is provided with the slant portions 94 to prevent the skirt portions from being hooked to the case with an increased certainty, the invention is not limited this way and permit variations without the slant portions.

The above connected clip packages 80, 100, 130, and 150 accommodate a series of clips comprising clips 12 used in the successive clipping device 10 but the invention is not limited this way. The clip package of the invention may accommodate a clip unit comprising a single clip used in the successive clipping device 10 and can still produce the same effects as described above.

Since the clipping device 10 has the configuration such that the clips are connected in a positional relationship twisted by 90° with respect to the adjacent ones and the connection rings are accordingly fitted to the clips in a positional relationship twisted by 90° with respect to the adjacent connection rings, the openings of the case are also formed in positions corresponding to the respective connection rings so as to be apart from each other by 90° in the circumferential direction. However, the invention is not limited this way. For example, where one uses a clip having a shape such that the claw portions are rotated by 90° with respect to the turned portion in the circumferential direction, i.e., a shape such that the claw portions 22, 22 are twisted by 90° with respect to the turned portion 24 in the circumferential direction, or where the clips and the connection rings are connected at the same angle, with connection members provided between the clips, the openings may be formed in the same circumferential direction because all the skirt portions are directed in the same direction.

Preferably, closed clips having a turned portion are used in the invention because clips of this type allow a biasing force to be generated upon pressing the turned portion, the biasing force acting upon the arm portions to expand. However, the invention may also be applied where open clips (U-shaped clips) without the turned portion are used.

While the clip package and the clip loading method of the present invention have been described in detail above, the examples given above should not be construed limitative, and various improvements and modifications are possible without departing from the spirit of the present invention. Further, the clipping device of the present invention using the clip package and the clip loading method of the invention is applicable not only to a soft endoscope but also to a hard endoscope.

## Claims

1. A clip package comprising:
a clip assembly including at least one endoscopic clip and at least one cylindrical connection ring connected to the at least one endoscopic clip, the connection ring having at least one skirt portion that is allowed to expand radially outwards and stick out when in natural state free from external force and closes inwards when depressed radially inwards,
a cylindrical case including an accommodation section for accommodating the clip assembly, the cylindrical case further including at least one opening in a position corresponding to the skirt portion of the connection ring of the clip assembly accommodated in the accommodation section, the at least one opening being provided to maintain the skirt portion in natural state, and
a cylindrical outer tube movably fitted over the outer periphery of the case and including at least one skirt closer for depressing and inwardly closing the skirt portion when the at least one skirt closer is moved to a position opposite the at least one opening.

2. A clip package according to Claim 1, wherein the clip assembly includes a series of clips comprising clips connected to each other such that a proximal end of a preceding clip is engaged with a forward end of a following clip and cylindrical connection rings covering joints of each adjacent preceding and following clips to maintain connection of the preceding and following clips.

3. A clip package according to Claim 1 or 2, wherein the at least one opening of the case is an aperture and the at least one skirt closer is a projection provided on the inner periphery of the outer tube.

4. A clip package according to Claim 1 or 2, wherein the at least one opening comprises a flap that, when free from external force, opens radially outwards along the at least one skirt portion in natural state and, when under external force, closes radially inwards, and wherein the at least one skirt closer depresses the flap radially inwards when the at least one skirt closer is moved to a position opposite the flap.

5. A clip package according to Claim 4, wherein the flap opens and closes with respect to the case by pivoting about an end side of the flap closer to the forward end of the case.

6. A clip package according to Claim 5, wherein the at least one skirt closer is moved to a position opposite the at least one opening by moving the outer tube from the forward end of the case toward the proximal end to cover the case.

7. A clip package according to Claim 4, wherein the flap opens and closes with respect to the case by pivoting about a circumferential end side of the flap.

8. A clip package according to Claim 7, wherein the at least one skirt closer is moved to a position opposite the at least one opening by turning the outer tube circumferentially with respect to the case.

9. A clip package according to any one of Claims 4 to 8, wherein the flap has a protuberance formed on its surface facing the at least one skirt portion.

10. A clip package according to any one of Claims 4 to 9, wherein the at least one skirt portion has a protuberance formed on its surface facing the at least one opening.

11. A clip package according to any one of Claims 1 to 10, wherein the outer tube comprises an aperture on a line extending in the direction in which the at least one skirt closer moves, the aperture being larger in area than the at least one opening.

12. A clip package according to any one of Claims 1 to 11, wherein the case has a slant portion formed on a plane opposite a proximal end of the at least one opening and radially widening from one end thereof closer to the proximal end of the case toward the at least one opening.

13. A clip package according to any one of Claims 1 to 12, wherein the at least one skirt portion of the at least one connection ring depresses and holds the at least one clip in the at least one connection ring when the at least one skirt portion is depressed radially and closed inwardly.

14. A clip package according to any one of Claims 1 to 13, wherein the case comprises at the proximal end thereof a recess for receiving a part of a sheath in which the clip assembly is to be loaded.

15. A method of loading a sheath for an endoscopic clipping device with the clip assembly accommodated in the case of the clip package according to any one of Claims 1 to 14, comprising the steps of:
connecting a forward end of a manipulating wire provided in the sheath for withdrawing the clip assembly to a connection member attached to a rearmost end of the clip assembly accommodated in the case,
connecting the forward end of the sheath and the proximal end of the case,
placing the case and the outer tube in their relative positions to cause the at least one skirt closer to close the at least one skirt portion, and
moving the manipulating wire and the sheath relative to each other in a direction in which the forward end of the manipulating wire moves toward the proximal end of the sheath so that the whole of the clip assembly is loaded in the sheath.
